# EUROPÄISCHE PATENTSCHRIFT

(11) **EP 0 805 798 B1**
(45) Veröffentlichungstag und Bekanntmachung des Hinweises auf die Patenterteilung: **05.01.2000**
(21) Anmeldenummer: 96900932.3
(22) Anmeldetag: 10.01.1996
(51) Int. Cl.: C07C 271/22, C07C 271/34, C07C 271/54, C07D 307/81, C07D 213/40, C07C 333/04, C07C 333/20, A01N 47/10, A01N 47/12, A01N 47/18, A01N 47/22

(54) **AMINOSÄUREAMID-DERIVATE UND IHRE VERWENDUNG ALS SCHÄDLINGSBEKÄMPFUNGSMITTEL**
AMINO ACID AMIDE DERIVATIVES AND THEIR USE AS PESTICIDES
DERIVES D'AMIDE D'AMINOACIDE ET LEUR UTILISATION COMME PESTICIDES

(30) Priorität: 23.01.1995 DE 19501841
(43) Veröffentlichungstag der Anmeldung: 12.11.1997
(73) Patentinhaber: BAYER AG, 51368 Leverkusen (DE)
(72) Erfinder: SEITZ, Thomas, D-40764 Langenfeld (DE); HÄNSSLER, Gerd, D-51381 Leverkusen (DE); STENZEL, Klaus, D-40595 Düsseldorf (DE)
(86) Internationale Anmeldenummer: EP9600071
(87) Internationale Veröffentlichungsnummer: WO9622970

(56) Entgegenhaltungen:
- EP-A- 0 398 072
- EP-A- 0 425 925
- EP-A- 0 554 729
- BULL. CHEM. SOC. JPN. (1993), 66(7), 2048-53 , XP002001309 SHIN, CHUGN GI ET AL: "Dehydrooligopeptides. XV. Useful syntheses of dehydrodipeptides by the enzymic coupling of.alpha.-dehydroglutamate with various.alpha.-amino acid amides using proteases"
- CHEMICAL ABSTRACTS, vol. 118, no. 21, 24.Mai 1993 Columbus, Ohio, US; abstract no. 212719, UEDA, Y. ET AL: "Synthesis of.alpha.-(S)-acylamino-N-(hydroxydioxocyc lobutenyl)-.beta.-lactams as potential antibiotics" XP002001314 & BIOORG. MED. CHEM. LETT. (1992), 2(12), 1541-6 ,
- CHEMICAL ABSTRACTS, vol. 110, no. 21, 22.Mai 1989 Columbus, Ohio, US; abstract no. 193407, SHIN, SHIGEKI ET AL: "A process for preparation of.alpha.,.beta.-dehydro-.alpha.-amino acid derivatives and peptides containing them" XP002001315 & JP,A,63 122 659 (SKI BAITSU K. K., JAPAN)
- BULL. CHEM. SOC. JPN. (1988), 61(7), 2687-9, XP002001310 YONEZAWA, YASUCHIKA ET AL: "Dehydrooligopeptides. IX. Syntheses and conversions of.alpha.-dehydroglutamine derivatives to N-carboxy-.alpha.- dehydroglutamine anhydride and.DELTA.1-dehydroglutaminyl dipeptides"
- BULL. CHEM. SOC. JPN. (1986), 59(11), 3573-9 , XP002001311 SHIN, CHUNG GI ET AL: "Dehydrooligopeptides. VII. Convenient synthesis of various dehydrodi- and tripeptide esters by using N-carboxy.alpha.-dehydroamino acid anhydride"
- BULL. CHEM. SOC. JPN. (1980), 53(10), 2905-9, XP002001312 YONEZAWA, YASUCHIKA ET AL: "Dehydrooligopeptides. I. The facile coupling of.alpha.-amino acids with.alpha.-dehydroamino acids to dehydrodipeptides"
- BULL. CHEM. SOC. JPN. (1995), 68(12), 3549-55 , XP002001313 SHIN, CHUNG-GI ET AL: "Dehydrooligopeptides. XVIII. Enzymic hydrolysis and coupling of dehydrodipeptide esters containing.alpha.-dehydroamino acid residue by using papain"

## Beschreibung

Die Erfindung betrifft neue substituierte Aminosäureamide, ein Verfahren zu ihrer Herstellung und ihre Venwendung als Schädlingsbekämpfungsmittel.

Es ist bekannt, daß bestimmte substituierte Aminosäureamide wie beispielsweise die Verbindung N-(t-Butyloxycarbonyl)-L-valin-[N-(1-phenylethyl)-amid] oder die Verbindung N-(t-Butyloxycarbonyl)-L-valin- {N-[1-(2-chlorphenyl)ethyl]-amid} oder die Verbindung N-(t-Butyloxycarbonyl)-L-valin-{N-methyl-N-[1-(4-chlorphenyl)-ethyl]-amid} fungizide Eigenschaften besitzen (vgl. z. B. EP 398 072). Weiterhin sind aus den Dokumenten Bull. Chem. Soc. Jpn. (1993), 66(7), S. 2050, Chemical Abstracts, Vol. 110, Nr. 21, 22. Mai, 1989, Bull. Chem. Soc, Jpn. (1988), 61(7), S. 2688, Bull. Chem. Soc. Jpn. (1986), 59(11), S. 3575 und Bull. Chem. Soc. Jpn. (1980), 53(10), S. 2907 Aminosäureamide bekannt. Eine fungizide Wirkung dieser Verbindungen ist bisher nicht beschrieben.

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden nun die neuen Aminosäureamide der allgemeinen Formel (I) gefunden, 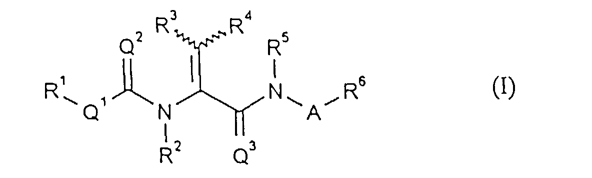 in welcher
- A: für eine Einfachbindung oder gegebenenfalls substituiertes Alkylen steht,
- Q¹, Q², Q³: gleich oder verschieden sind und für Sauerstoff oder Schwefel stehen,
- R¹: für jeweils gegebenenfalls substituiertes Alkyl, Alkenyl, Alkinyl, Cycloalkyl, Cycloalkenyl, Arylalkyl, Aryl, Heterocyclylalkyl oder Heterocyclyl steht,
- R², R⁵: gleich oder verschieden sind und jeweils für Wasserstoff oder jeweils gegebenenfalls substituiertes Alkyl stehen,
- R³: für Wasserstoff, jeweils gegebenenfalls substituiertes Alkyl oder Cycloalkyl steht,
- R⁴: für jeweils gegebenenfalls substituiertes Alkyl oder Cycloalkyl steht, oder
- R³ und R⁴: gemeinsam mit dem Kohlenstoffatom, an das sie gebunden sind einen gegebenenfalls substituierten carbocyclischen Ring bilden und
- R⁶: für jeweils gegebenenfalls substituiertes Cyloalkyl, Cycloalkenyl, Aryl oder Heterocyclyl steht.

Ausgenommen sind die Verbindungen

Cbz―ΔGlu(OMe)―Phe―NHPh

Cbz―ΔGlu(OMe)―Try―NHPh

Cbz―ΔGlu(OMe)―NHPh

Cbz―ΔGln(Bu^{t})―Phe―OMe

Boc―ΔnVal(OMe)―L-Tyr―OMe

Cbz―ΔBut(OMe)―Phe―OEt

Cbz―ΔBut(OMe)―Tyr―OMe

Cbz-ΔBut(OMe)―Trp―OMe

und

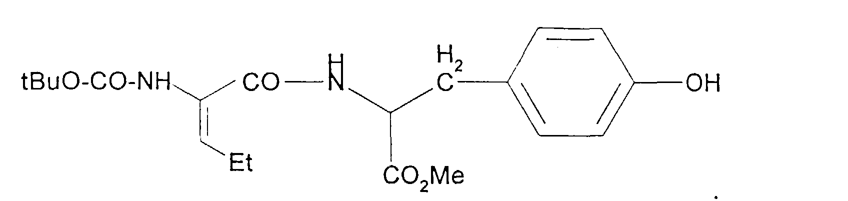

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Aryl steht für aromatische, mono oder polycyclische Kohlenwasserstoffringe, wie z.B. Phenyl, Naphthyl, Anthranyl, Phenanthryl, vorzugsweise Phenyl oder Naphthyl, insbesondere Phenyl.

Heterocyclyl steht für gesättigte oder ungesättigte, sowie aromatische, ringförmige Verbindungen, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Gegebenenfalls bilden die ringförmigen Verbindungen mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden.

Cycloalkenyl steht für carbocyclische, ringförmige Verbindungen, die mindestens eine Doppelbindung enthalten und gegebenenfalls mit weiteren carbocyclischen, ankondensierten oder überbrückten Ringen ein polycyclisches Ringsystem bilden.

Weiterhin wurde gefunden, daß man die neuen Aminosäureamide der allgemeinen Formel (1) erhält, wenn man N-substituierte Aminosäuren der allgemeinen Formel (II), 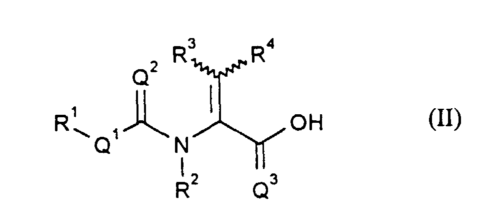 in welcher
Q¹, Q², Q³, R¹, R², R³ und R⁴ die oben angegebene Bedeutung haben,
mit Aminen der allgemeinen Formel (III), 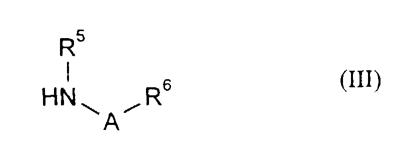 in welcher
A, R⁵ und R⁶ die oben angegebene Bedeutung haben,
gegebenenfalls in Gegenwart eines Reaktionshilfsmittels und gegebenenfalls in Gegenwart eines Verdünnungsmittels, umsetzt.

Schließlich wurde gefunden, daß die neuen Aminosäureamide der allgemeinen Formel (I) sehr starke fungizide Wirkung zeigen.

Die erfindungsgemäßen Verbindungen liegen gegebenenfalls als Mischungen verschiedener möglicher isomerer Formen, insbesondere von Stereoisomeren, wie z.B. E- und Z-, threo- und erythro-, sowie optischen Isomeren vor. Es werden sowohl die E- als auch die Z-Isomeren, wie auch die threo- und erythro-, sowie die optischen Isomeren sowie beliebige Mischungen dieser Isomeren beansprucht.

Gegenstand der Erfindung sind vorzugsweise Verbindungen der Formel (I), in welcher
- A: für eine Einfachbindung oder für gegebenenfalls einfach oder mehrfach, gleich oder verschieden substituiertes Alkylen mit 1 bis 6 Kohlenstoffatomen steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, substituiertes Aryl oder Heterocyclyl.
- Q¹, Q², Q³: gleich oder verschieden sind und jeweils für Sauerstoff oder Schwefel stehen,
- R¹: für gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 8 Kohlenstoffatomen;
für jeweils gegebenenfalls durch Halogen substituiertes Alkenyl oder Alkinyl mit jeweils bis zu 8 Kohlenstoffatomen;
für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxycarbonyl substituiertes Cycloalkyl oder Cycloalkenyl mit 3 bis 7 Kohlenstoffatomen;
für jeweils gegebenenfalls einfach oder mehrfach, gleich oder verschieden substituiertes Aryl, Arylalkyl oder für Heterocyclyl mit 3 bis 12 Ringgliedern steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio steht.
- R², R⁵: gleich oder verschieden sind und jeweils für Wasserstoff oder für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 4 Kohlenstoffatomen oder Cycloalkyl mit 3 bis 6 Kohlenstoffatomen stehen,
- R³: für Wasserstoff, für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 8 Kohlenstoffatomen;
für jeweils gegebenenfalls durch Halogen substituiertes Alkenyl oder Alkinyl mit jeweils bis zu 8 Kohlenstoffatomen;
für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxycarbonyl substituiertes Cycloalkyl mit 3 bis 6 Kohlenstoffatomen steht,
- R⁴: für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 8 Kohlenstoffatomen;
für jeweils gegebenenfalls durch Halogen substituiertes Alkenyl oder Alkinyl mit jeweils bis zu 8 Kohlenstoffatomen;
für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxycarbonyl substituiertes Cycloalkyl mit 3 bis 6 Kohlenstoffatomen steht, oder
- R³ und R⁴: gemeinsam mit dem Kohlenstoffatom, an das sie gebunden sind einen gegebenenfalls einfach oder mehrfach, gleich oder verschieden durch Halogen, Cyano, Hydroxy, Amino, C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituierten carbocyclischen Ring mit 3 bis 8 Kohlenstoffatomen bilden und
- R⁶: für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxycarbonyl substituiertes Cycloalkyl oder Cycloalkenyl mit 3 bis 8 Kohlenstoffatomen;
für jeweils gegebenenfalls einfach oder mehrfach, gleich oder verschieden substituiertes Aryl oder für Heterocyclyl mit 3 bis 12 Ringgliedern steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

Die Erfindung betrifft insbesondere Verbindungen der Formel (I), in welcher
- A: für eine Einfachbindung oder für gegebenenfalls einfach oder mehrfach, gleich oder verschieden durch Fluor, Chlor, Cyano, Methoxy, Phenyl, Tolyl, Ethylphenyl, Methoxyphenyl, Ethoxyphenyl, Chlorphenyl, Fluorphenyl, Trifluormethylphenyl oder Xylyl substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- Q¹, Q², Q³: gleich oder verschieden sind und jeweils für Sauerstoff oder Schwefel stehen,
- R¹: für gegebenenfalls einfach bis fünffach durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-, 2-, 3-(3-Methylpentyl), 2-Ethylbutyl, 1-, 3-, 4-(2,2-Dimetylbutyl), 1-, 2-(2,3-Dimethylbutyl,
für jeweils gegebenenfalls einfach bis dreifach durch Fluor, Chlor oder Brom substituiertes Allyl, Crotonyl, 1-Methyl-allyl, Propargyl oder 1-Methyl-propargyl;
für jeweils gegebenenfalls einfach bis sechsfach durch Fluor, Chlor, Brom, Methoxy, Ethoxy, Cyano, Carboxy, Phenyl (welches gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiert ist), Methyl, Ethyl, n- oder i-Propyl, Methoxy-carbonyl oder Ethoxy-carbonyl substituiertes Cyclopropyl, Cyclobutyl, Cyclopentyl oder Cyclohexyl;
für jeweils gegebenenfalls einfach bis dreifach substituiertes Phenyl, Naphthyl, Benzyl, Phenethyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
   Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl , Methylamino, Ethylamino, n- oder i-Propylamino, Dimethylamino, Diethylamino, Acetyl, Propionyl, Acetyloxy, Methoxy-carbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Methylendioxy oder Ethylendioxy,
Cyclopropyl, Cyclobutyl, Cyclopentyl oder Cyclohexyl.
- R², R⁵: gleich oder verschieden sind und jeweils für Wasserstoff oder für Methyl, Ethyl, n- oder i-Propyl, n-, i-, s- oder t-Butyl stehen,
- R³: für Wasserstoff oder für jeweils gegebenenfalls einfach bis fünffach durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls einfach bis dreifach durch Fluor, Chlor oder Brom substituiertes Allyl, Crotonyl, 1-Methyl-allyl, Propargyl oder 1-Methyl-propargyl;
für jeweils gegebenenfalls einfach bis sechsfach durch Fluor, Chlor, Brom, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht,
- R⁴: für jeweils gegebenenfalls einfach bis fünffach durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls einfach bis dreifach durch Fluor, Chlor oder Brom substituiertes Allyl, Crotonyl, 1-Methyl-allyl, Propargyl oder 1-Methyl-propargyl;
für jeweils gegebenenfalls einfach bis sechsfach durch Fluor, Chlor, Brom, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht, oder
- R³ und R⁴: gemeinsam mit dem Kohlenstoffatom, an das sie gebunden sind für gegebenenfalls einfach bis sechsfach durch Fluor, Chlor, Brom, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl stehen und
- R⁶: für jeweils gegebenenfalls einfach bis sechsfach durch Fluor, Chlor, Brom, Cyano, Carboxy, Methyl, Ethyl, Methoxy, Ethoxy, n- oder i-Propyl, Methoxy-carbonyl oder Ethoxy-carbonyl substituiertes Cyclopropyl, Cyclobutyl, Cyclopentyl oder Cyclohexyl;
für jeweils gegebenenfalls einfach bis dreifach substituiertes Phenyl, Naphthyl, Benzyl, Phenethyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl , Methylamino, Ethylamino, n- oder i-Propylamino, Dimethylamino, Diethylamino, Acetyl, Propionyl, Acetyloxy, Methoxy-carbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Methylendioxy oder Ethylendioxy,
Cyclopropyl, Cyclobutyl, Cyclopentyl oder Cyclohexyl.

Besonders bevorzugt sind Verbindungen der Formel (I), in welcher
- A: für eine Einfachbindung oder für gegebenenfalls einfach oder mehrfach, gleich oder verschieden durch Fluor, Chlor, Cyano, Methoxy, Phenyl, Tolyl, Ethylphenyl, Methoxyphenyl, Ethoxyphenyl, Chlorphenyl, Fluorphenyl, Trifluormethylhenyl oder Xylyl substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- Q¹, Q², Q³: gleich oder verschieden sind und jeweils für Sauerstoff oder Schwefel stehen,
- R¹: für gegebenenfalls einfach bis dreifach durch Fluor, Chlor, Brom, Methoxy, Ethoxy, Methylthio, Ethylthio, (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls einfach bis dreifach durch Fluor oder Chlor substituiertes Allyl, Crotonyl, 1-Methyl-allyl, Propargyl oder 1-Methyl-propargyl;
für jeweils gegebenenfalls einfach bis sechsfach durch Fluor, Chlor, Brom, Methoxy, Ethoxy, Phenyl (welches gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiert ist), Methyl, Ethyl, n- oder i-Propyl substituiertes Cyclobutyl, Cyclopentyl oder Cyclohexyl;
für jeweils gegebenenfalls einfach bis dreifach substituiertes Phenyl, Naphthyl, Benzyl, Furyl, Benzofuranyl, Thienyl, Benzothienyl, Pyridyl, Chinolyl, Tetrahydrofuryl oder Perhydropyranyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-, i-, s-oder t-Butyl, Methoxy, Ethoxy, n- oder i-Propoxy, Methylthio, Ethylthio, n-oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Methylendioxy oder Ethylendioxy,
- R², R⁵: gleich oder verschieden sind und jeweils für Wasserstoff oder für Methyl stehen,
- R³: für Wasserstoff oder für jeweils gegebenenfalls einfach bis fünffach durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls einfach bis dreifach durch Fluor, Chlor oder Brom substituiertes Allyl, Crotonyl, 1-Methyl-allyl, Propargyl oder 1-Methyl-propargyl;
für jeweils gegebenenfalls einfach bis sechsfach durch Fluor, Chlor, Brom, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht,
- R⁴: für jeweils gegebenenfalls einfach bis fünffach durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls einfach bis dreifach durch Fluor, Chlor oder Brom substituiertes Allyl, Crotonyl, 1-Methyl-allyl, Propargyl oder 1-Methyl-propargyl;
für jeweils gegebenenfalls einfach bis sechsfach durch Fluor, Chlor, Brom, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht,
- R³ und R⁴: gemeinsam mit dem Kohlenstoffatom, an das sie gebunden sind für gegebenenfalls einfach bis sechsfach durch Fluor, Chlor, Brom, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl stehen und
- R⁶: für jeweils gegebenenfalls einfach bis sechsfach durch Fluor, Chlor, Brom, Cyano, Carboxy, Methyl, Ethyl, Methoxy, Ethoxy, n- oder i-Propyl, Methoxy-carbonyl oder Ethoxy-carbonyl substituiertes Cyclopropyl, Cyclobutyl, Cyclopentyl oder Cyclohexyl;
für jeweils gegebenenfalls einfach bis dreifach substituiertes Phenyl, Naphthyl, Benzyl, Phenethyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethyl sulfinyl oder Trifluormethylsulfonyl , Methylamino, Ethylamino, n- oder 1-Propylamino, Dimethylamino, Diethylamino, Acetyl, Propionyl, Acetyloxy, Methoxy-carbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Methylendioxy oder Ethylendioxy,
Cyclopropyl, Cyclobutyl, Cyclopentyl oder Cyclohexyl.

Eine ganz besonders bevorzugte Gruppe erfindungsgemäßer Verbindungen sind diejenigen Verbindungen der Formel (I), in welcher
- A: für eine Einfachbindung oder für Methylen, 1,1-Ethylen, 1,2-Ethylen, 1,1-Propylen, 1,2-Propylen oder 2,2-Propylen steht,
- Q¹, Q², Q³: für Sauerstoff stehen,
- R¹: für Methyl, Ethyl, n- oder i-Propyl, n-, i-, s- oder t-Butyl, Cyclopentyl, Cyclohexyl oder für gegebenenfalls durch Methyl, Ethyl, Methoxy, Ethoxy, Brom, Chlor oder Fluor substituiertes Phenyl oder Benzyl steht,
- R², R⁵: für Wasserstoff stehen,
- R³: für Wasserstoff, Methyl, Ethyl oder Cyclopropyl steht,
- R⁴: für Methyl, Ethyl oder Cyclopropyl steht, oder
- R³ und R⁴: gemeinsam mit dem Kohlenstoffatom, an das sie gebunden sind einen Cyclopentan- oder Cyclohexanring bilden und
- R⁶: für jeweils gegebenenfalls einfach oder zweifach durch Methyl, Ethyl, Methoxy oder Ethoxy substituiertes Cyclohexyl;
für jeweils gegebenenfalls einfach bis dreifach substituiertes Phenyl, Naphthyl, Furyl, Benzofuranyl, Pyrrolyl, Indolyl, Thienyl, Benzothienyl, Pyridyl, Chinolyl, Pyrimidyl, Pyridazinyl, Pyrazinyl steht, wobei die möglichen Substituenten vorzugsweise aus der nachstehenden Aufzählung ausgewählt sind:
   Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl , Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Methylendioxy oder Ethylendioxy.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Die zur Durchführung des erfindungsgemäßen Verfahrens als Ausgangsstoffe benötigten Aminosäurederivate sind durch die Formel (II) allgemein definiert. In dieser Formel (II) haben Q¹, Q², Q³, R¹, R², R³ und R⁴ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Q¹, Q², Q³, R¹, R², R³ und R⁴ angegeben wurden.

Die Ausgangsstoffe der Formel (II) sind bekannt und/oder können nach an sich bekannten Verfahren hergestellt werden (vgl. z. B. Shin, Chung Gi; et. al. Chem. Pharm. Bull. 1984, 3934-3944).

Die zur Durchführung des erfindungsgemäßen Verfahrens weiterhin als Ausgangsstoffe benötigten Amine sind durch die Formel (III) allgemein definiert. In dieser Formel (III) haben A, R⁵ und R⁶ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für A, R⁵ und R⁶ angegeben wurden.

Die Amine der allgemeinen Formel (III) sind bekannte Reagenzien in der organischen Chemie.

Das erfindungsgemäße Verfahren wird gegebenenfalls in Gegenwart eines Verdünnungsmittels durchgeführt. Als Verdünnungsmittel zur Durchführung des erfindungsgemäßen Verfahrens kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid oder Sufone, wie Sulfolan.

Das erfindungsgemäße Verfahren wird gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ) oder Triphenylphosphin/Tetrachlorkohlenstoff.

Das erfindungsgemäße Verfahren wird gegebenenfalls in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung des erfindungsgemäßen Verfahrens in einem größeren Bereich variiert werden. Im allgemeinen arbeitet man bei Temperaturen zwischen -78°C und +120°C, vorzugsweise bei Temperaturen zwischen -60°C und +25°C.

Zur Durchführung des erfindungsgemäßen Verfahrens zur Herstellung der Verbindungen der Formel (I) setzt man pro Mol des Aminosäurederivates der Formel (II) im allgemeinen 0,5 bis 5 Mol, vorzugsweise äquimolare Mengen an Amin der Formel (III) ein.

Die Reaktionsdurchführung, Aufarbeitung und Isolierung der Reaktionsprodukte erfolgt nach bekannten Verfahren (vgl. die Herstellungsbeispiele).

Das erfindungsgemäße Verfahren kann auch als zweistufiger Prozess durchgeführt werden. Dabei weden die Aminosäurederivate der allgemeinen Formel (II) zunächst in eine aktivierte Form überführt und in einem anschließenden Schritt mit den Aminen der allgemeinen Formel (III) zu den erfindungsgemäßen Aminosäureamiden der allgemeinen Formel (I) umgesetzt.

Als aktivierte Form der Aminosäurederivate der Formel (II) kommen alle Carboxy-aktivierten Derivate infrage, wie z.B. Säurehalogenide, bevorzugt Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxisuccinimidester sowie Addukte mit Kondensationsmitteln, wie z.B. Dicyclohexylcarbodiimid oder in situ erzeugte aktivierte Formen der Aminosäuren.

Zur Durchführung des zweistufigen Verfahrens werden vorzugsweise die den Aminosäuren der Formel (II) entsprechenden Säurechloride und gemischten Anhydride eingesetzt. Sie können hergestellt werden, indem man die Aminosäuren der Formel (II) oder deren Salze mit einem Halogenierungsmittel oder einem der allgemein bekannten Mittel zur Herstellung von gemischten Anhydriden, wie beispielsweise Phosphorpentachlorid, Thionylchlorid, Oxalylchlorid oder Chlorameisensäureester, in allgemein bekannter Art und Weise umsetzt.

Auch die beiden Stufen des zweistufigen Prozesses werden jeweils gegebenenfalls in Gegenwart eines Verdünnungsmittels und gegebenenfalls in Gegenwart eines Katalysators durchgeführt. Als Verdünnungsmittel und Katalysatoren kommen vorzugsweise diejenigen Verdünnungsmittel und Katalysatoren infrage, die bereits im Zusammenhang mit der Beschreibung des einstufigen Verfahrens angegeben wurden.

Die Reaktionsdurchführung, Aufarbeitung und Isolierung der Reaktionsprodukte erfolgt nach bekannten Verfahren.
Die erfindungsgemäßen Wirkstoffe weisen eine starke mikrobizide Wirkung auf und werden zur Bekämpfung von unerwünschten Mikroorganismen praktisch eingesetzt. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen, erlaubt eine Behandlung von oberirdischen Pflanzenteilen, sowie auch eine Behandlung von Pflanz- und Saatgut und des Bodens.

Dabei werden die erfindungsgemäßen Wirkstoffe mit besonders gutem Erfolg zur Bekämpfung von Krankheiten im Wein-, Obst- und Gemüseanbau, wie beispielsweise gegen Plasmopara- und Phytophthora-Arten eingesetzt.

Die Wirkstoffe werden in Abhängigkeit von ihren jeweiligen physikalischen und/oder chemischen Eigenschaften gegebenenfalls in übliche Formulierungen übergeführt, wie z. B. Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatsche Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es werden in den Formulierungen gegebenenfalls Haftmittel wie Carboxymethylcellulose, natürliche und synthetische, pulverige, körnige oder latexförmige Polymere verwendet, wie z.B. Gummiarabicum, Polyvinylalkohol, Polyvinylacetat, sowie natürliche Phospholipide, wie Kephaline und Lecithine und synthetische Phospholipide. Weitere mögliche Additive sind mineralische und vegetabile Öle.

Gegebenenfalls werden Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink zugesetzt.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe werden als solche oder in ihren Formulierungen auch in Mischung mit bekannten Fungiziden, Bakteriziden, Akariziden, Nematiziden oder Insektiziden verwendet, um so z.B. das Wirkungsspektrum zu verbreitern oder Resistenzentwicklungen vorzubeugen.

In vielen Fällen werden dabei synergistische Wirkungen beobachtet.

Für die Mischungen kommen beispielsweise infrage:
**Fungizide:**
   2-Aminobutan; 2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Di-chloro-N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyanophenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb,
   Cymoxanil, Cyproconazole, Cyprofuram,
   Dichlorophen, Diclobutrazol, Diclofluanid, Diclomezin, Dicloran, Diethofencarb,
   Difenoconazol, Dimethirimol, Dimethomorph, Diniconazol, Dinocap, Diphenylamin, Dipyrithion, Ditalimfos, Dithianon, Dodin, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropi-morph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
   Guazatine,
   Hexachlorobenzol, Hexaconazol, Hymexazol,
   Imazalil, Imibenconazol, Iminoctadin, Iprobenfos (IBP), Iprodion, Isoprothiolan,
   Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
   Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickeldimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram
**Bakterizide:**
   Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
   Cadusafos, Carbaryl, Carbofuran, Carbophenothion, Carbosulfan, Cartap, CGA 157 419, CGA 184 699, Chloethocarb, Chlorethoxyfos, Chloretoxyfos, Chlorfenvinphos, Chlorfluazuron, Chlormephos, Chlorpyrifos, Chlorpyrifos M, Cis-Resmethnn, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etofenprox, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox, Imidacloprid, Iprobenfos, Isazophos, Isofenphos, Isoprocarb, Isoxathion, Ivemectin,
   Lamda-cyhalothrin, Lufenuron,
   Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram
   Omethoat, Oxamyl, Oxydemethon M, Oxydeprofos,
   Parathion A, Parathion M, Permethrin, Phenthoat, Phorat, Phosalon, Phosmet, Phosphamdon, Phoxim, Pirimicarb, Pirimiphos M, ,Primiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
   Quinalphos,
   RH 5992,
   Salithion, Sebufos, Silafluofen, Sulfotep, Sulprofos,
   Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, YI 5301/5302, Zetamethrin.

Gegebenenfalls werden die erfindungsgemäßen Wirkstoffe auch mit anderen bekannten Wirkstoffen, wie Herbiziden oder auch mit Düngemitteln und Wachstumsregulatoren gemischt.
Die Wirkstoffe werden als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet. Die Anwendung geschieht in üblicher Weise, z.B. durch Gießen, Verspritzen, Versprühen, Verstreuen, Verstäuben, Verschäumen, Bestreichen usw. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele

### Beispiel 1:

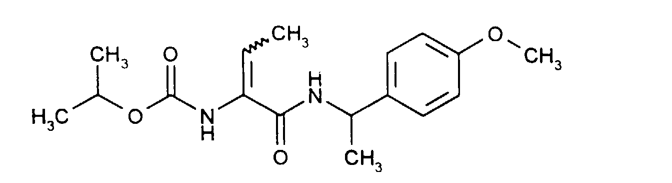

Zu 1,0g (0,005 mol) 2-(i-Propoxycarbonylamino)-but-2-ensäure in 10 ml Dichlormethan gibt man bei -20°C 0,5 g (0,005 Mol) N-Methylpiperidin und anschließend tropfenweise unter Rühren bei -20°C 0,7 g (0,005 Mol) Chlorameisensäureisobutylester, rührt nach beendeter Zugabe weitere 10 Minuten bei -20°C und gibt 0,75 g (0,005 Mol) 1-(4-Methoxyphenyl)-ethylamin zu, wobei die Temperatur -15°C nicht überschreiten soll. Anschließend rührt man weitere 2 Stunden bei -15°C und 15 Stunden bei Raumtemperatur, filtriert den ausgefallenen Feststoff ab, wäscht mit Dichlormethan nach, engt das Filtrat im Vakuum ein, nimmt den Rückstand in Wasser auf, extrahiert mehrfach mit Essigsäureethylester, wäscht die vereinigten organischen Phasen mit wäßriger Natriumhydrogencarbonatlösung und Wasser, trocknet über Natriumsulfat und entfernt das Lösungsmittel im Vakuum. Man erhält 0,8 g (47 % der Theorie) an N-[1-(4-Methoxyphenyl)-ethyl]-2-(i-propoxycarbonylamino)-but-2-ensäureamid als farbloses Öl. ¹H-NMR (CDCl₃, TMS), δ (ppm): 1,25 (m, 6 H); 1,50 (d, 3 H); 1,74 (d, 3 H); 3,79 (s, 3 H).

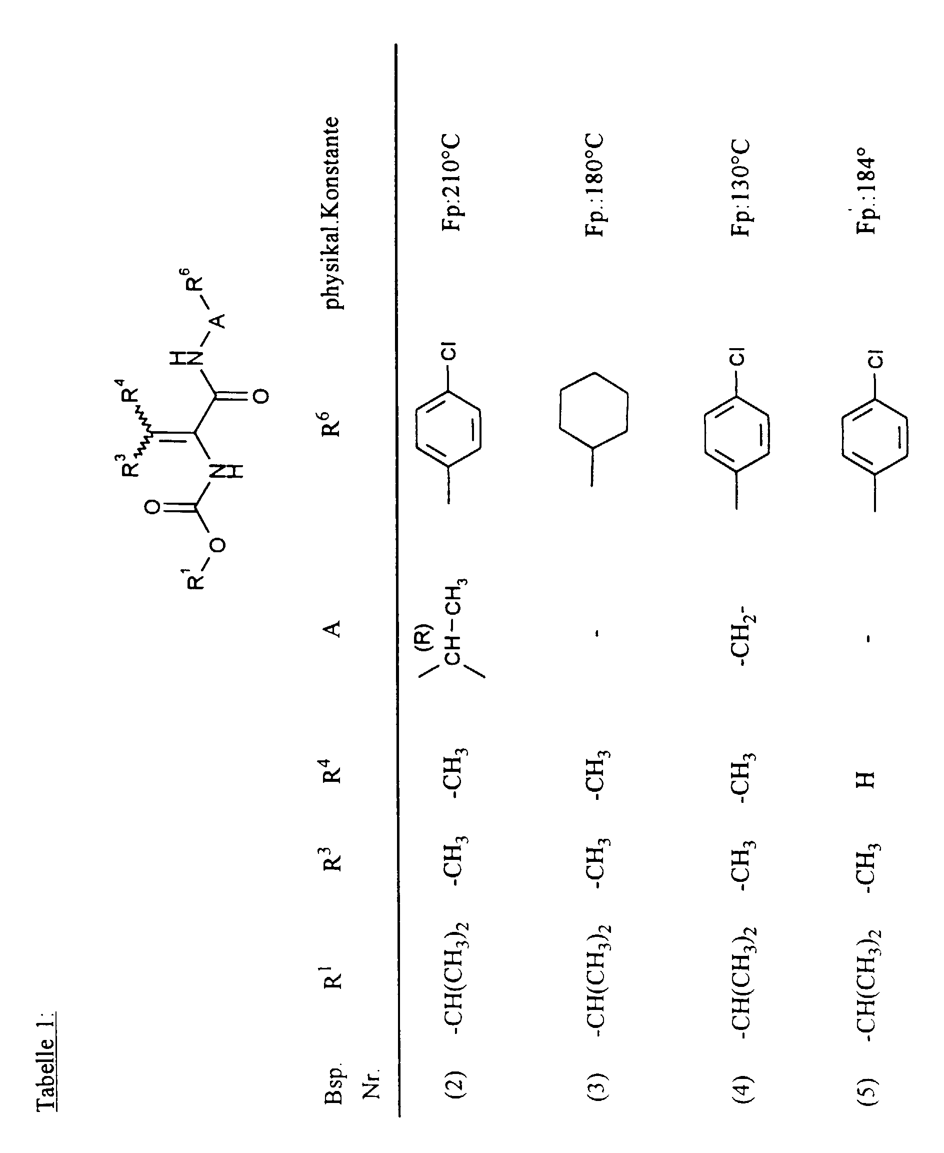

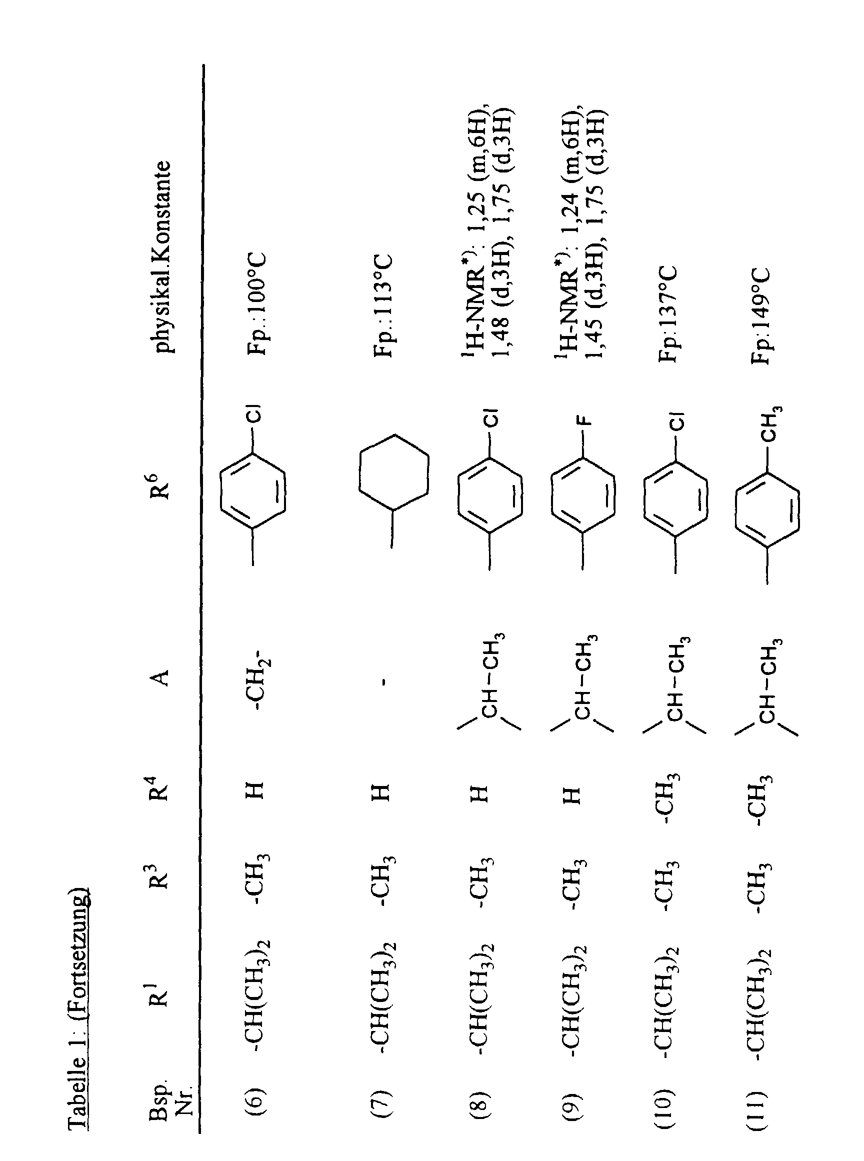

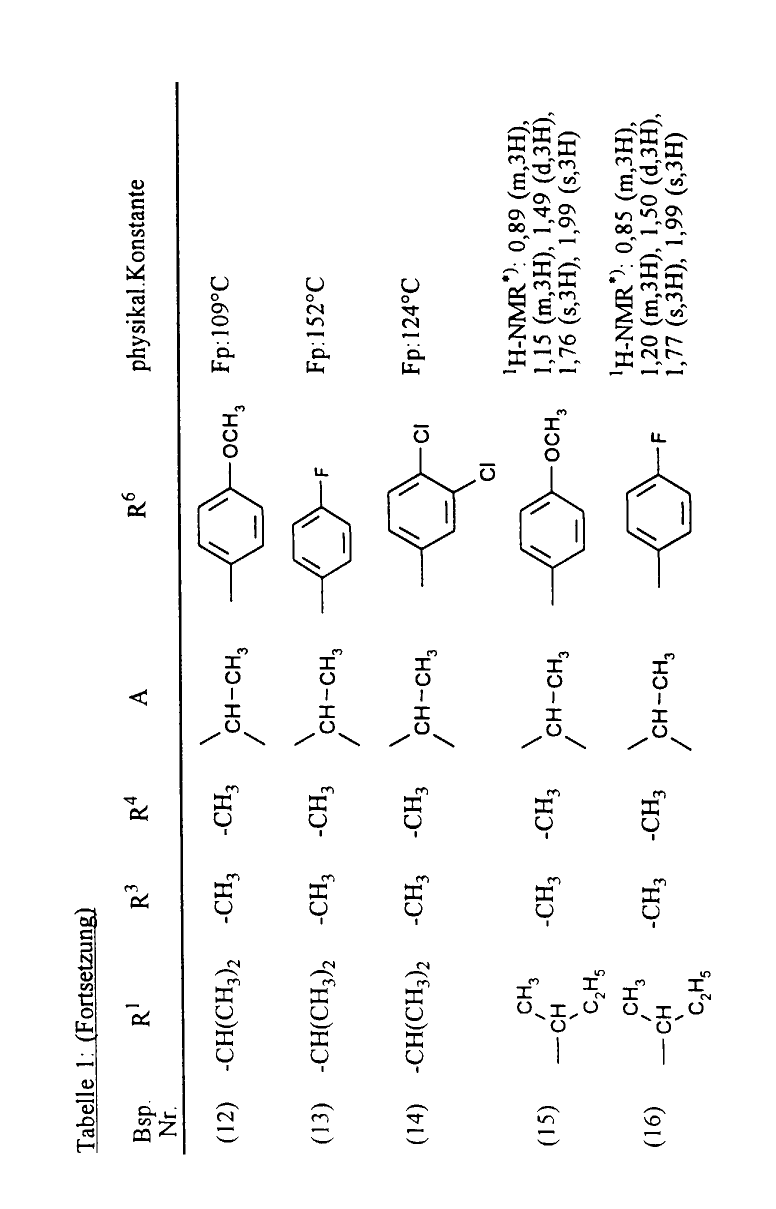

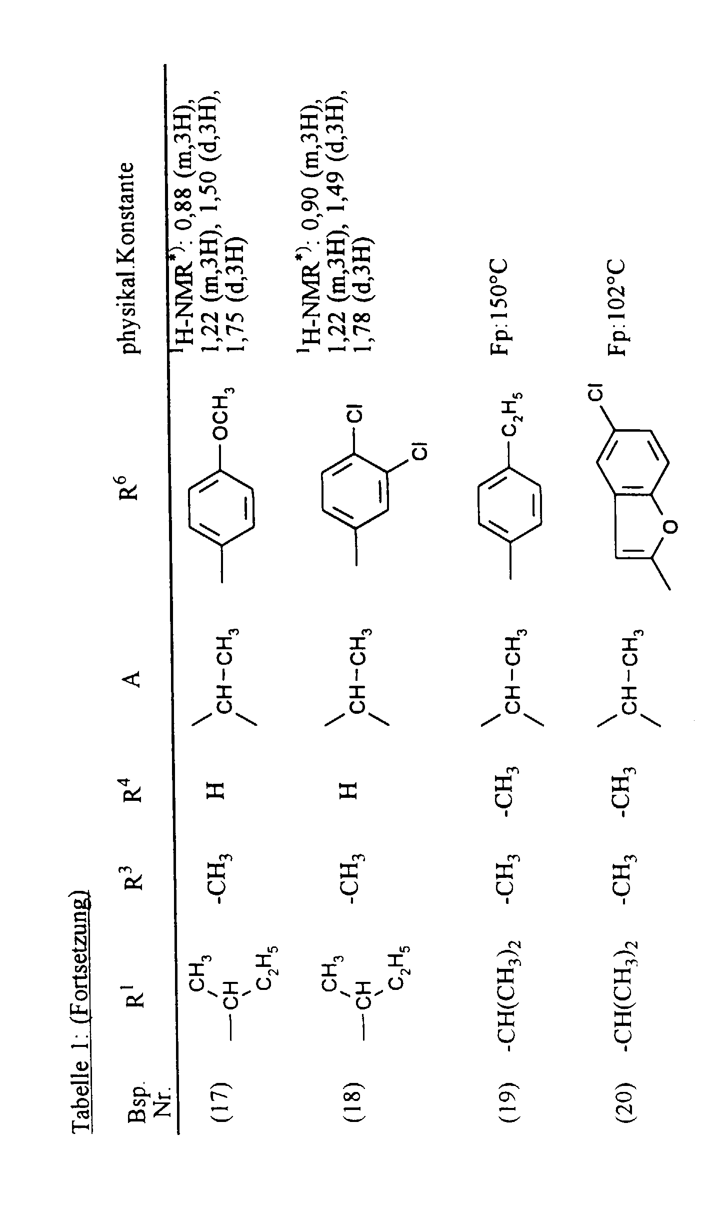

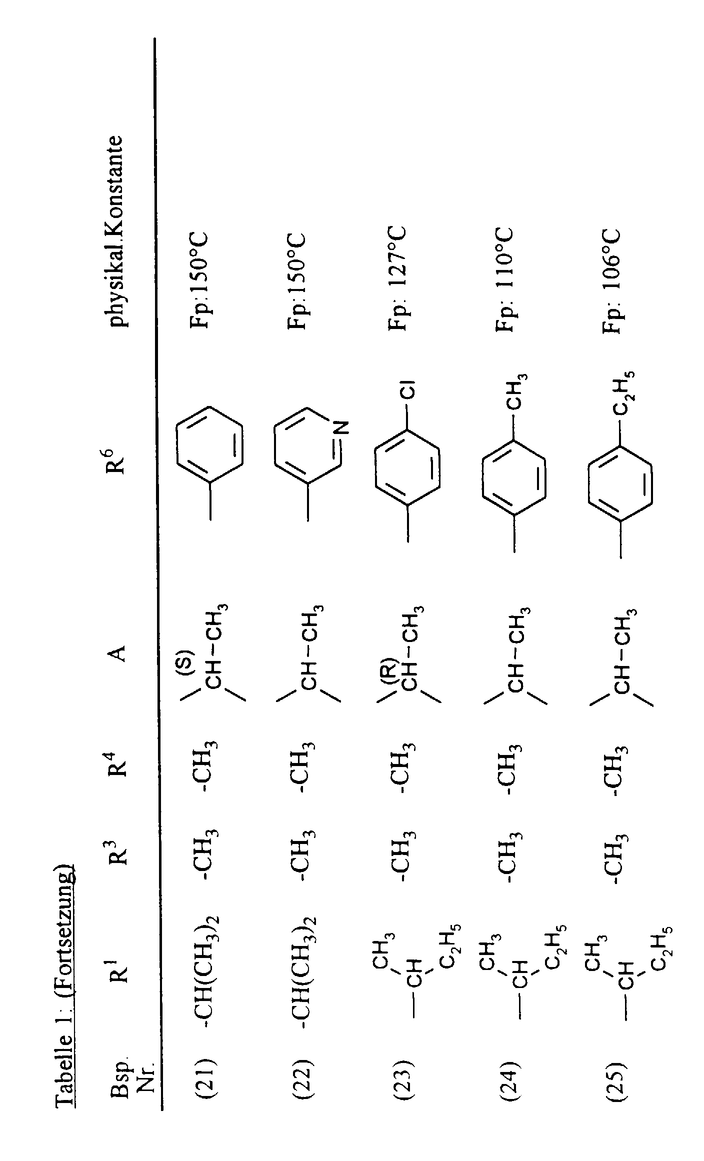

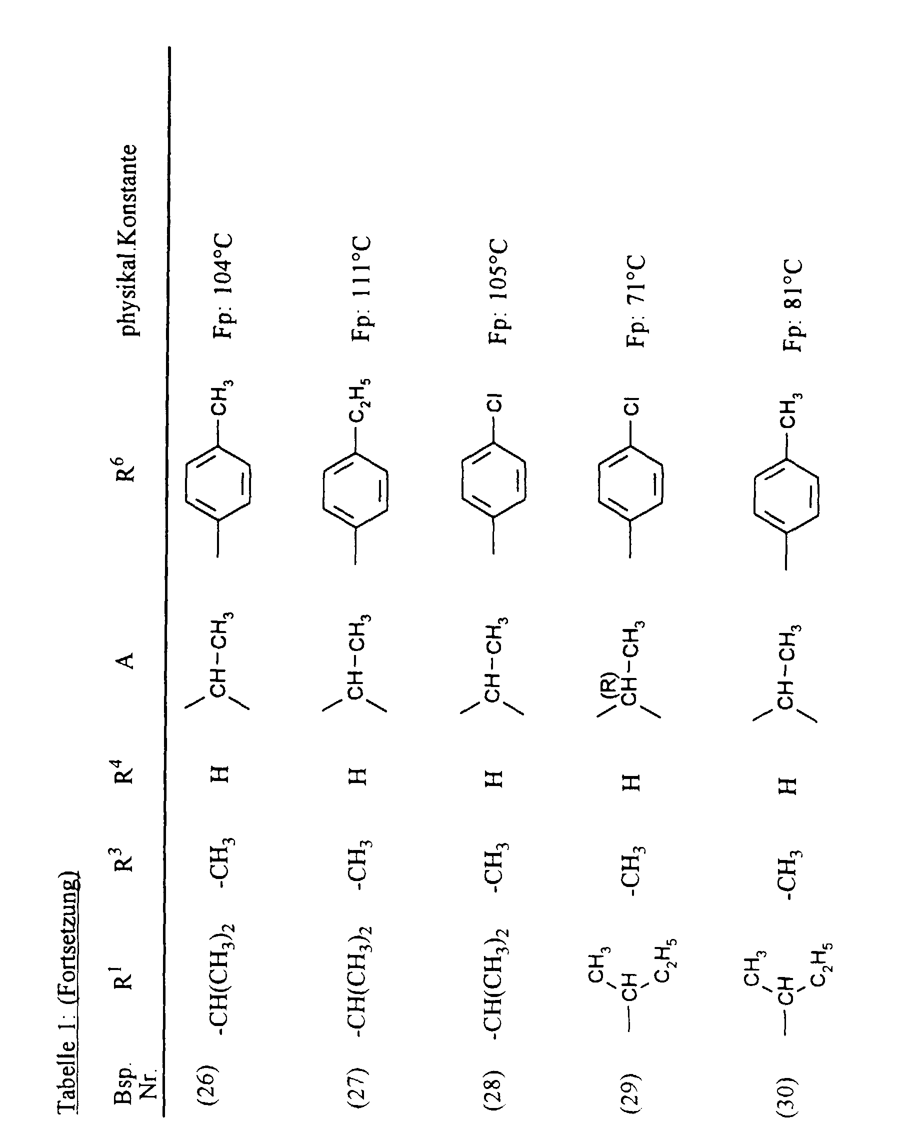

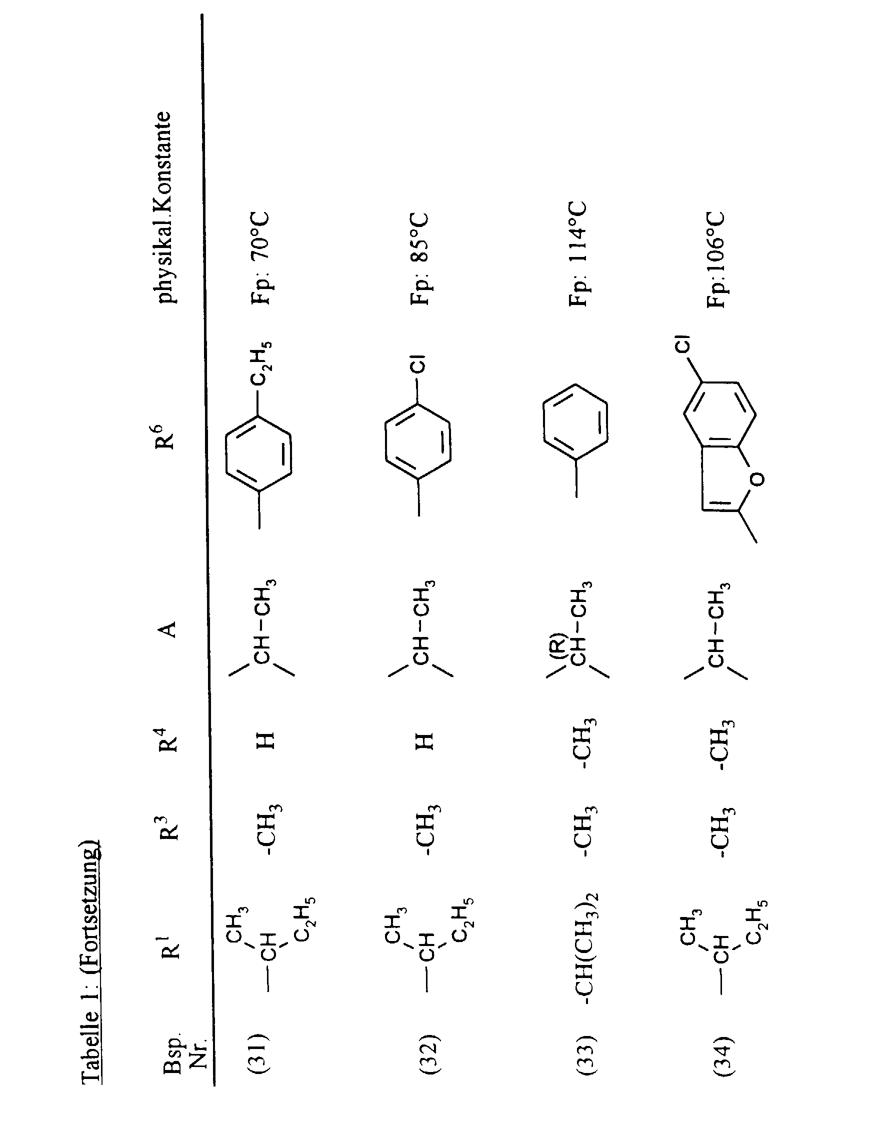

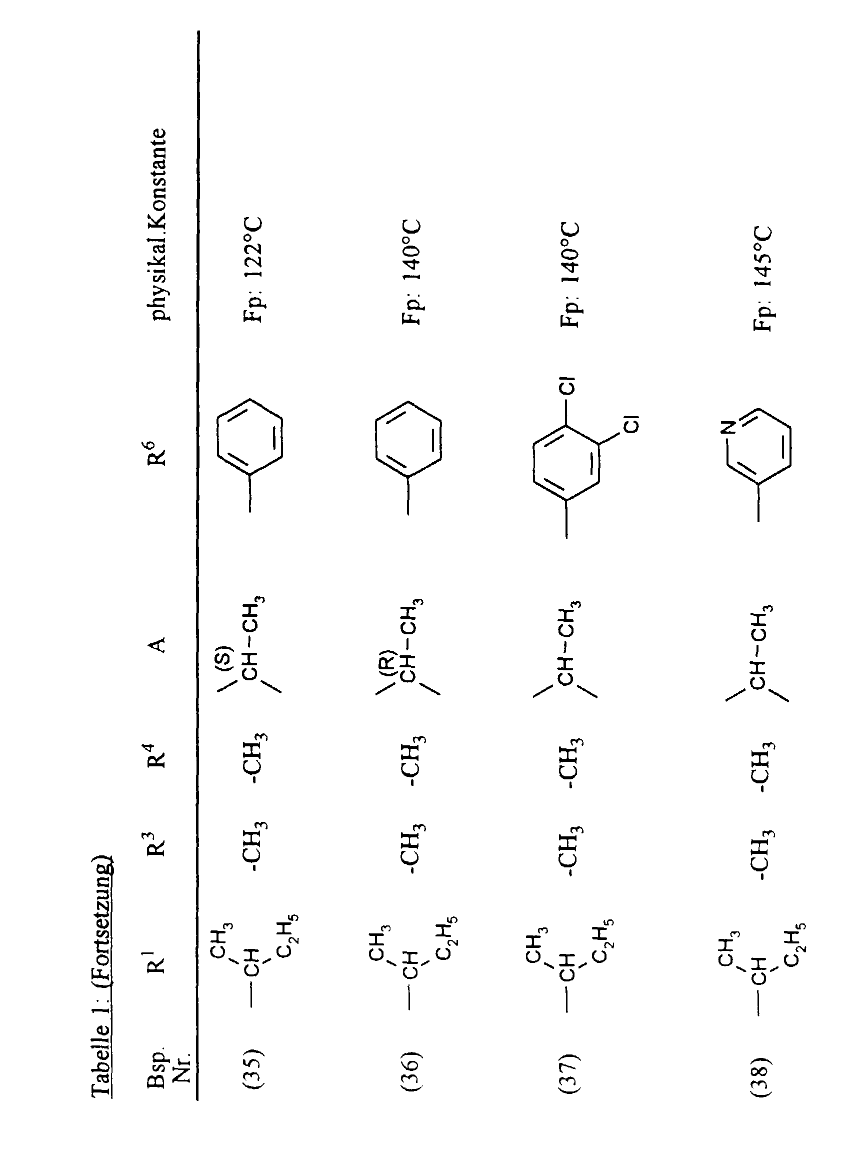

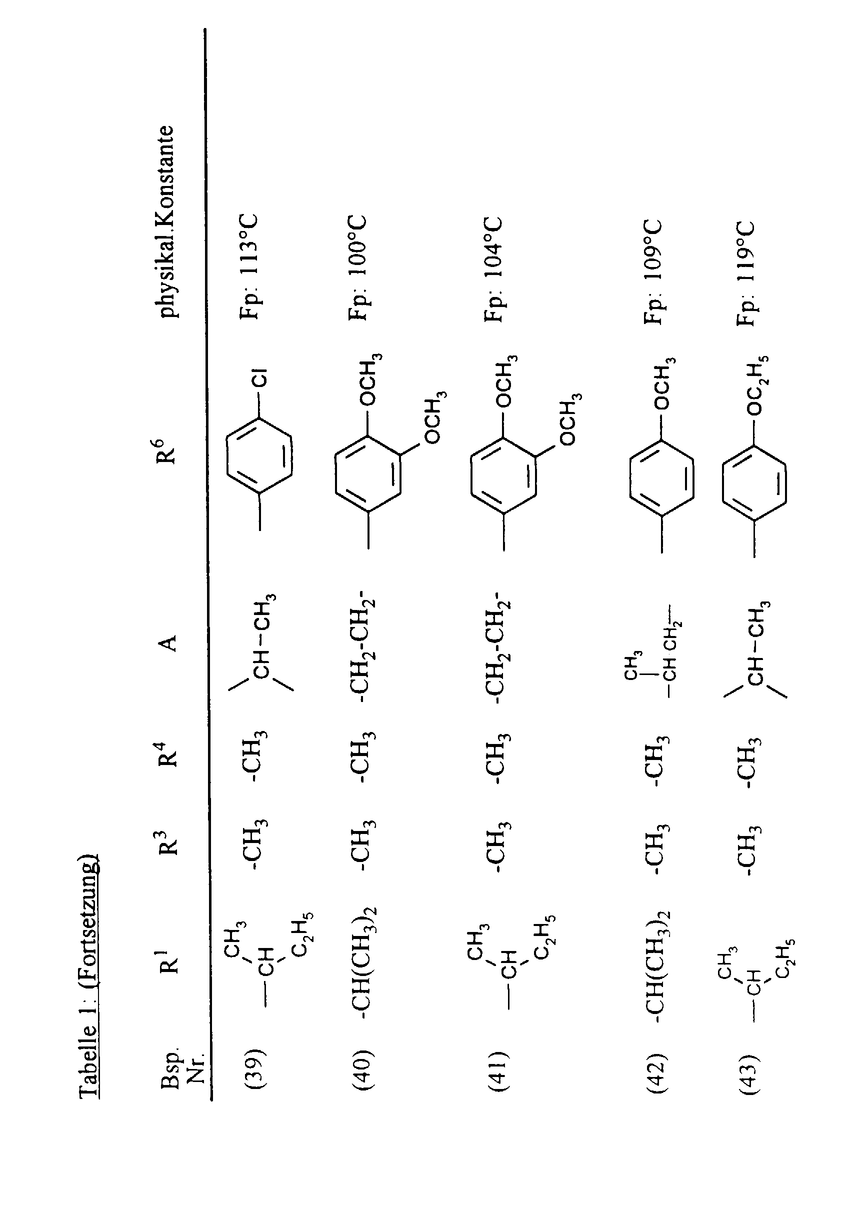

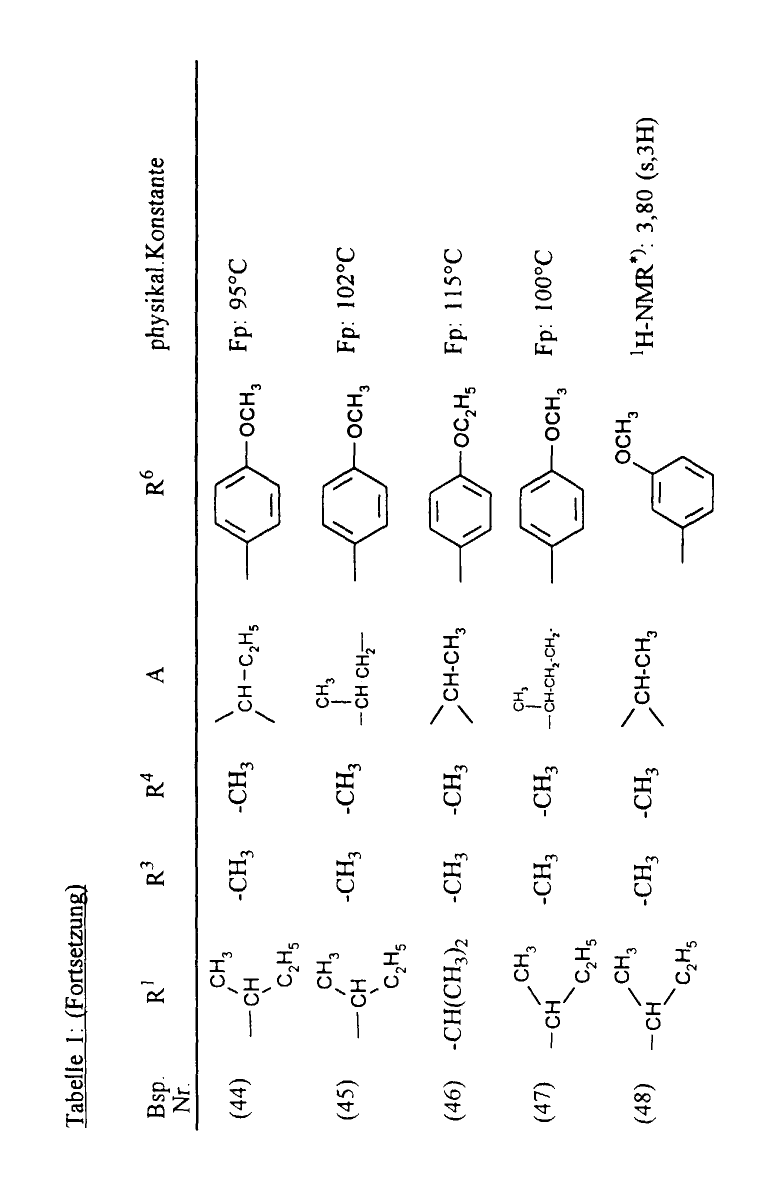

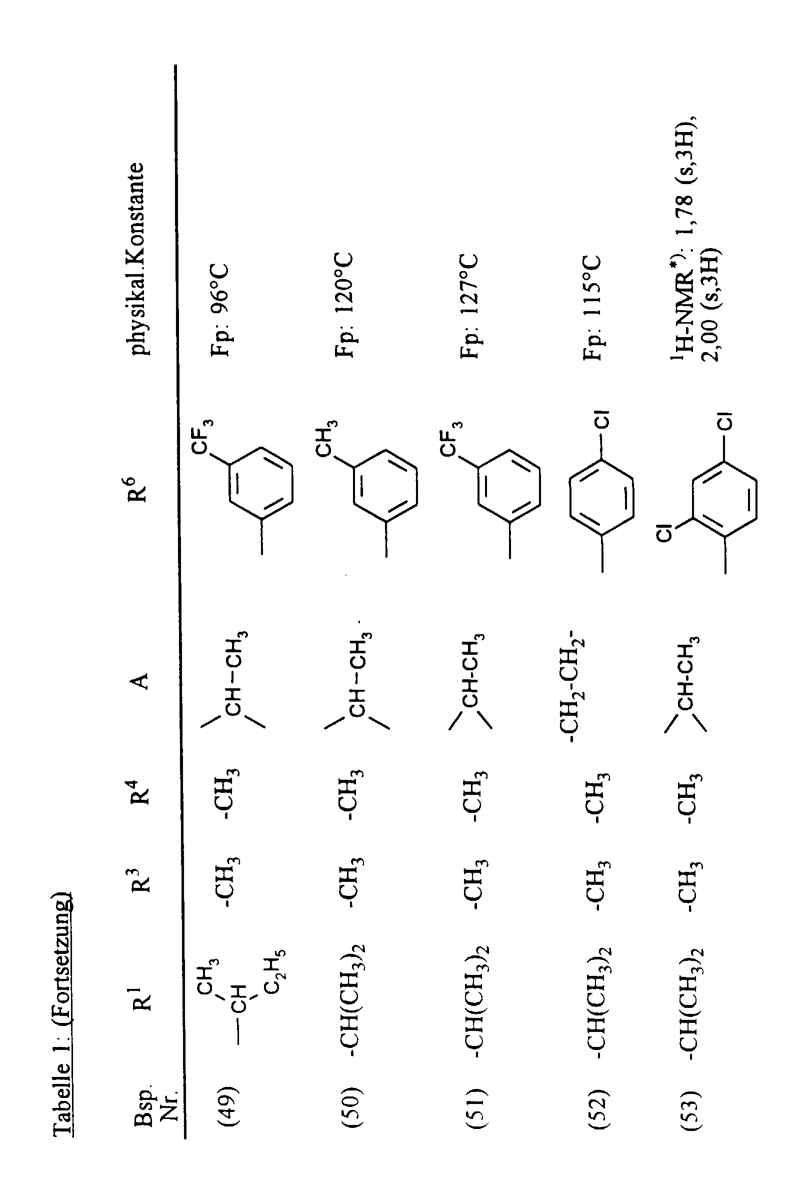

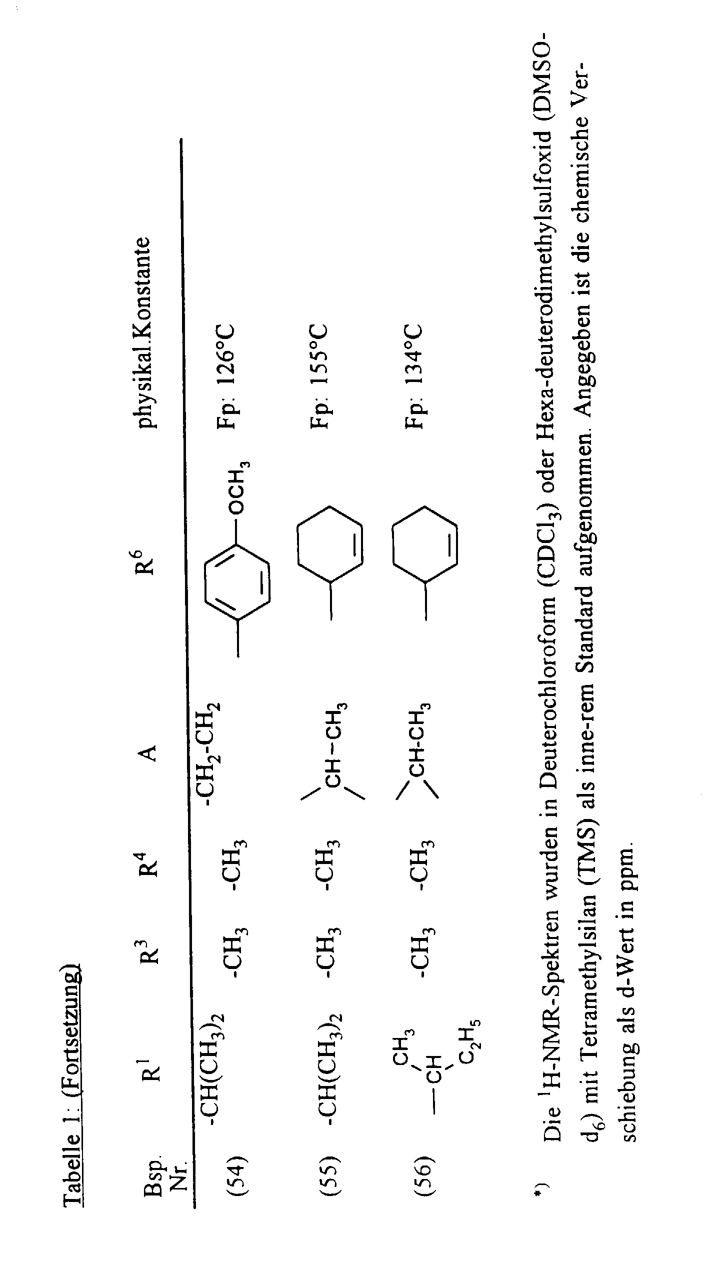

### Anwendungsbeispiele:

### Beispiel: A

**Plasmopara-Test** (Reben) / protektiv
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100% relativer Luftfeuchtigkeit. Anschließend werden die Pflanzen 5 Tage im Gewächshaus bei 21°C und ca. 90% Luftfeuchtigkeit aufgestellt. Die Pflanzen werden dann angefeuchtet und 1 Tag in eine Feuchtkammer gestellt.

6 Tage nach der Inokulation erfolgt die Auswertung.

Bei diesem Test zeigt z.B. die folgende Verbindung (2), (8), (10), (11), (12), (15), (19), (20), (23), (36), (39) und (41) bei einer Wirkstoffkonzentration von 100ppm einen Wirkungsgrad von über 90 %.

### Beispiel B

**Phytophthora-Test** (Tomate) / protektiv
Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

4 Tage nach der Inokulation erfolgt die Auswertung.

Bei diesem Test zeigt z.B. die folgende Verbindung (2) der Herstellungsbeispiele bei einer Wirkstoffkonzentration von 0,05 % einen Wirkungsgrad von 100 %.

## Patentansprüche

1. Verbindungen der Formel (I) 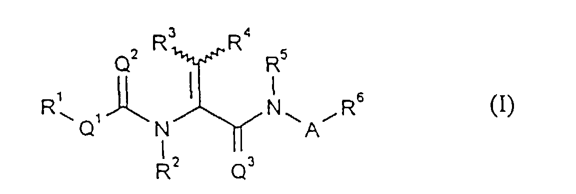 in welcher
A für eine Einfachbindung oder gegebenenfalls substituiertes Alkylen steht,
Q¹, Q², Q³ gleich oder verschieden sind und für Sauerstoff oder Schwefel stehen,
R¹ für jeweils gegebenenfalls substituiertes Alkyl, Alkenyl, Alkinyl, Cycloalkyl, Cycloalkenyl, Arylalkyl, Aryl, Heterocyclylalkyl oder Heterocyclyl steht,
R², R⁵ gleich oder verschieden sind und jeweils für Wasserstoff oder jeweils gegebenenfalls substituiertes Alkyl stehen,
R³ für Wasserstoff, jeweils gegebenenfalls substituiertes Alkyl oder Cycloalkyl steht,
R⁴ für jeweils gegebenenfalls substituiertes Alkyl oder Cycloalkyl steht, oder
R³ und R⁴ gemeinsam mit dem Kohlenstoffatom, an das sie gebunden sind einen gegebenenfalls substituierten carbocyclischen Ring bilden und
R⁶ für jeweils gegebenenfalls substituiertes Cycloalkyl, Cycloalkenyl, Aryl oder Heterocyclyl steht,
ausgenommen sind die Verbindungen
Cbz―ΔGlu(OMe)―Phe―NHPh
Cbz―ΔGlu(OMe)―Try―NHPh
Cbz―ΔGlu(OMe)―NHPh
Cbz―ΔGln(Bu^{t})―Phe―OMe
Boc―ΔnVal(OMe)―L-Tyr―OMe
Cbz―ΔBut(OMe)―Phe―OEt
Cbz―ΔBut(OMe)―Tyr―OMe
Cbz―ΔBut(OMe)―Trp―OMe
und

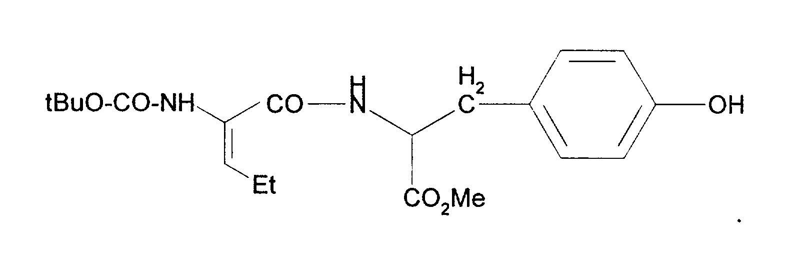

2. Verbindungen der Formel (I) gemäß Anspruch I, in welcher
A für eine Einfachbindung oder für gegebenenfalls einfach oder mehrfach, gleich oder verschieden substituiertes Alkylen mit 1 bis 6 Kohlenstoffatomen steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, substituiertes Aryl oder Heterocyclyl.
Q¹, Q², Q³ gleich oder verschieden sind und jeweils für Sauerstoff oder Schwefel stehen,
R¹ für gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 8 Kohlenstoffatomen;
für jeweils gegebenenfalls durch Halogen substituiertes Alkenyl oder Alkinyl mit jeweils bis zu 8 Kohlenstoffatomen;
für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substituiertes Cycloalkyl oder Cycloalkenyl mit 3 bis 7 Kohlenstoffatomen;
für jeweils gegebenenfalls einfach oder mehrfach, gleich oder verschieden substituiertes Aryl, Arylalkyl oder für Heterocyclyl mit 3 bis 12 Ringgliedern steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio steht.
R², R⁵ gleich oder verschieden sind und jeweils für Wasserstoff oder für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 4 Kohlenstoffatomen oder Cycloalkyl mit 3 bis 6 Kohlenstoffatomen stehen,
R³ für Wasserstoff, für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 8 Kohlenstoffatomen;
für jeweils gegebenenfalls durch Halogen substituiertes Alkenyl oder Alkinyl mit jeweils bis zu 8 Kohlenstoffatomen;
für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substituiertes Cycloalkyl mit 3 bis 6 Kohlenstoffatomen steht,
R⁴ für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 8 Kohlenstoffatomen; für jeweils gegebenenfalls durch Halogen substituiertes Alkenyl oder Alkinyl mit jeweils bis zu 8 Kohlenstoffatomen;
für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substituiertes Cycloalkyl mit 3 bis 6 Kohlenstoffatomen steht, oder
R³ und R⁴ gemeinsam mit dem Kohlenstoffatom, an das sie gebunden sind einen gegebenenfalls einfach oder mehrfach, gleich oder verschieden durch Halogen, Cyano, Hydroxy, Amino, C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituierten carbocyclischen Ring mit 3 bis 8 Kohlenstoffatomen bilden und
R⁶ für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substituiertes Cycloalkyl oder Cycloalkenyl mit 3 bis 8 Kohlenstoffatomen;
für jeweils gegebenenfalls einfach oder mehrfach, gleich oder verschieden substituiertes Aryl oder für Heterocyclyl mit 3 bis 12 Ringgliedern steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils
zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

3. Verbindungen der Formel (I)gemäß Anspruch 1, in welcher
A für eine Einfachbindung oder für gegebenenfalls einfach oder mehrfach, gleich oder verschieden durch Fluor, Chlor, Cyano, Methoxy, Phenyl, Tolyl, Ethylphenyl, Methoxyphenyl, Ethoxyphenyl, Chlorphenyl, Fluorphenyl, Trifluormethylphenyl oder Xylyl substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
Q¹, Q², Q³ gleich oder verschieden sind und jeweils für Sauerstoff oder Schwefel stehen,
R¹ für gegebenenfalls einfach bis fünffach durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-, 2-, 3-(3-Methylpentyl), 2-Ethylbutyl, 1-, 3-, 4-(2,2-Dimetylbutyl), 1-, 2-(2,3-Dimethylbutyl,
für jeweils gegebenenfalls einfach bis dreifach durch Fluor, Chlor oder Brom substituiertes Allyl, Crotonyl, 1-Methyl-allyl, Propargyl oder 1-Methyl-propargyl;
für jeweils gegebenenfalls einfach bis sechsfach durch Fluor, Chlor, Brom, Methoxy, Ethoxy, Cyano, Carboxy, Phenyl (welches gegebenenfalls durch Fluor, Chlor, Brom, Cyano, Methyl, Ethyl, n-oder i-Propyl, n-, i-, s- oder t-Butyl, Trifluormethyl, Methoxy, Ethoxy, n- oder i-Propoxy, Difluormethoxy oder Trifluormethoxy substituiert ist), Methyl, Ethyl, n- oder i-Propyl, Methoxy-carbonyl oder Ethoxy-carbonyl substituiertes Cyclopropyl, Cyclobutyl, Cyclopentyl oder Cyclohexyl;
für jeweils gegebenenfalls einfach bis dreifach substituiertes Phenyl, Naphthyl, Benzyl, Phenethyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl , Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Methylendioxy oder Ethylendioxy,
Cyclopropyl, Cyclobutyl, Cyclopentyl oder Cyclohexyl.
R², R⁵ gleich oder verschieden sind und jeweils für Wasserstoff oder für Methyl, Ethyl, n- oder i-Propyl, n-, i-, s- oder t-Butyl stehen,
R³ für Wasserstoff oder für jeweils gegebenenfalls einfach bis fünffach durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls einfach bis dreifach durch Fluor, Chlor oder Brom substituiertes Allyl, Crotonyl, 1-Methyl-allyl, Propargyl oder 1-Methyl-propargyl;
für jeweils gegebenenfalls einfach bis sechsfach durch Fluor, Chlor, Brom, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht,
R⁴ für jeweils gegebenenfalls einfach bis fünffach durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls einfach bis dreifach durch Fluor, Chlor oder Brom substituiertes Allyl, Crotonyl, 1-Methyl-allyl, Propargyl oder 1-Methyl-propargyl;
für jeweils gegebenenfalls einfach bis sechsfach durch Fluor, Chlor, Brom, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht, oder
R³ und R⁴ gemeinsam mit dem Kohlenstoffatom, an das sie gebunden sind für gegebenenfalls einfach bis sechsfach durch Fluor, Chlor, Brom, Cyano, Carboxy, Methyl, Ethyl, n- oder 1-Propyl, Methoxy-carbonyl oder Ethoxy-carbonyl substituiertes Cyclopropyl, Cyclobutyl, Cyclopentyl oder Cyclohexyl stehen und
R⁶ für jeweils gegebenenfalls einfach bis sechsfach durch Fluor, Chlor, Brom, Cyano, Carboxy, Methyl, Ethyl, Methoxy, Ethoxy, n- oder i-Propyl, Methoxy-carbonyl oder Ethoxy-carbonyl substituiertes Cyclopropyl, Cyclobutyl, Cyclopentyl oder Cyclohexyl;
für jeweils gegebenenfalls einfach bis dreifach substituiertes Phenyl, Naphthyl, Benzyl, Phenethyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Methylendioxy oder Ethylendioxy,
Cyclopropyl, Cyclobutyl, Cyclopentyl oder Cyclohexyl.

4. Verbindungen der Formel (I) gemäß Anspruch 1, in welcher
A für eine Einfachbindung oder für gegebenenfalls einfach oder mehrfach, gleich oder verschieden durch Fluor, Chlor, Cyano, Methoxy, Phenyl, Tolyl, Ethylphenyl, Methoxyphenyl, Ethoxyphenyl, Chlorphenyl, Fluorphenyl, Trifluormethylhenyl oder Xylyl substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
Q¹, Q², Q³ gleich oder verschieden sind und jeweils für Sauerstoff oder Schwefel stehen,
R¹ für gegebenenfalls einfach bis dreifach durch Fluor, Chlor, Brom, Methoxy, Ethoxy, Methylthio, Ethylthio, (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls einfach bis dreifach durch Fluor oder Chlor substituiertes Allyl, Crotonyl, 1-Methyl-allyl, Propargyl oder 1-Methyl-propargyl;
für jeweils gegebenenfalls einfach bis sechsfach durch Fluor, Chlor, Brom, Methoxy, Ethoxy, Phenyl (welches gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiert ist), Methyl, Ethyl, n- oder i-Propyl substituiertes Cyclobutyl, Cyclopentyl oder Cyclohexyl;
für jeweils gegebenenfalls einfach bis dreifach substituiertes Phenyl, Naphthyl, Benzyl, Furyl, Benzofuranyl, Thienyl, Benzothienyl, Pyridyl, Chinolyl, Tetrahydrofuryl oder Perhydropyranyl steht, wobei die möglichen Substituenten aus der nachstehenden Aufzählung ausgewählt sind:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Methylendioxy oder Ethylendioxy,
R², R⁵ gleich oder verschieden sind und jeweils für Wasserstoff oder für Methyl stehen,
R³ für Wasserstoff oder für jeweils gegebenenfalls einfach bis fünffach durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls einfach bis dreifach durch Fluor, Chlor oder Brom substituiertes Allyl, Crotonyl, 1-Methyl-allyl, Propargyl oder 1-Methyl-propargyl;
für jeweils gegebenenfalls einfach bis sechsfach durch Fluor, Chlor, Brom, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht,
R⁴ für jeweils gegebenenfalls einfach bis fünffach durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl,
für jeweils gegebenenfalls einfach bis dreifach durch Fluor, Chlor oder Brom substituiertes Allyl, Crotonyl, 1-Methyl-allyl, Propargyl oder 1-Methyl-propargyl;
für jeweils gegebenenfalls einfach bis sechsfach durch Fluor, Chlor, Brom, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht,
R³ und R⁴ gemeinsam mit dem Kohlenstoffatom, an das sie gebunden sind für gegebenenfalls einfach bis sechsfach durch Fluor, Chlor, Brom, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl stehen und
R⁶ für jeweils gegebenenfalls einfach bis sechsfach durch Fluor, Chlor, Brom, Cyano, Carboxy, Methyl, Ethyl, Methoxy, Ethoxy, n- oder i-Propyl, Methoxy-carbonyl oder Ethoxy-carbonyl substituiertes Cyclopropyl, Cyclobutyl, Cyclopentyl oder Cyclohexyl;
für jeweils gegebenenfalls einfach bis dreifach substituiertes Phenyl, Naphthyl, Benzyl, Phenethyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Methylendioxy oder Ethylendioxy,
Cyclopropyl, Cyclobutyl, Cyclopentyl oder Cyclohexyl.

5. Verbindungen der Formel (I) gemäß Anspruch 1, in welcher
A für eine Einfachbindung oder für Methylen, 1,1-Ethylen, 1,2-Ethylen, 1,1-Propylen, 1,2-Propylen oder 2,2-Propylen steht,
Q¹, Q², Q³ für Sauerstoff stehen,
R¹ für Methyl, Ethyl, n- oder i-Propyl, n-, i-, s- oder t-Butyl, Cyclopentyl, Cyclohexyl oder für gegebenenfalls durch Methyl, Ethyl, Methoxy, Ethoxy, Brom, Chlor oder Fluor substituiertes Phenyl oder Benzyl steht,
R², R⁵ für Wasserstoff stehen,
R³ für Wasserstoff, Methyl, Ethyl oder Cyclopropyl steht,
R⁴ für Methyl, Ethyl oder Cyclopropyl steht, oder
R³ und R⁴ gemeinsam mit dem Kohlenstoffatom, an das sie gebunden sind einen Cyclopentan- oder Cyclohexanring bilden und
R⁶ für jeweils gegebenenfalls einfach oder zweifach durch Methyl, Ethyl, Methoxy oder Ethoxy substituiertes Cyclohexyl;
für jeweils gegebenenfalls einfach bis dreifach substituiertes Phenyl, Naphthyl, Furyl, Benzofuranyl, Pyrrolyl, Indolyl, Thienyl, Benzothienyl, Pyridyl, Chinolyl, Pyrimidyl, Pyridazinyl, Pyrazinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl , Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Methylendioxy oder Ethylendioxy.

6. Schädlingsbekämpfungsmittel, gekennzeichnet durch einen Gehalt an mindestens einer Verbindung der Formel (I) nach Anspruch 1.

7. Verfahren zur Bekämpfung von Schädlingen, dadurch gekennzeichnet, daß man Verbindungen der Formel (I) nach Anspruch 1 auf Schädlinge und/oder ihren Lebensraum einwirken läßt.

8. Verwendung von Verbindungen der Formel (I) nach den Ansprüchen 1 bis 5 zur Bekämpfung von Schädlingen.

9. Verfahren zur Herstellung von Schädlingsbekämpfungsmitteln, dadurch gekennzeichnet, daß man Verbindungen der Formel (I) nach den Ansprüchen 1 bis 5 mit Streckmitteln und/oder oberflächenaktiven Mitteln vermischt.

10. Verfahren zur Herstellung von Verbindungen der Formel (I) nach Anspruch 1, dadurch gekennzeichnet, daß man N-substituierte Aminosäuren der allgemeinen Formel (II), 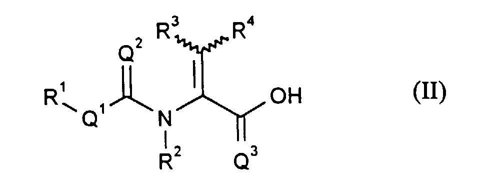 in welcher
Q¹, Q², Q³, R¹, R², R³ und R⁴ die in Anspruch 1 angegebenen Bedeutungen haben,
mit Aminen der allgemeinen Formel (III), 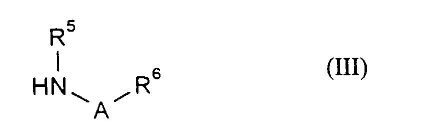 in welcher
A, R⁵ und R⁶ die in Anspruch 1 angegebenen Bedeutungen haben, gegebenenfalls in Gegenwart eines Reaktionshilfsmittels und gegebenenfalls in Gegenwart eines Verdünnungsmittels, umsetzt.

## Claims

1. Compounds of the formula (I) 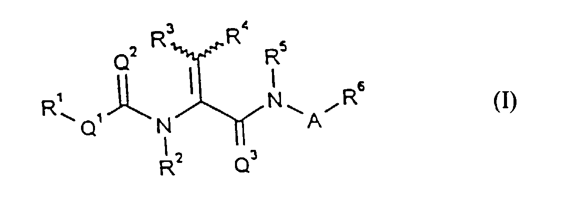 in which
A represents a single bond or optionally substituted alkylene,
Q¹, Q², Q³ are identical or different and each represent oxygen or sulphur,
R¹ represents respectively optionally substituted alkyl, alkenyl, alkinyl, cycloalkyl, cycloalkenyl, arylalkyl, aryl, heterocyclylalkyl or heterocyclyl,
R², R⁵ are identical or different and each represent hydrogen or respectively optionally substituted alkyl,
R³ represents hydrogen, or respectively optionally substituted alkyl or cycloalkyl,
R⁴ represents respectively optionally substituted alkyl or cycloalkyl, or
R³ and R⁴ together with the carbon atom that they are attached to form an optionally substituted carbocyclic ring and
R⁶ represents respectively optionally substituted cycloalkyl, cycloalkenyl, aryl or heterocyclyl,
with the exception of the compounds
Cbz―ΔGlu(OMe)―Phe―NHPh
Cbz―ΔGlu(OMe)―Try―NHPh
Cbz―ΔGlu(OMe)―NHPh
Cbz―ΔGln(Bu^{t})―Phe―OMe
Boc―ΔnVal(OMe)―L-Tyr―OMe
Cbz―ΔBut(OMe)―Phe―OEt
Cbz―ΔBut(OMe)―Tyr―OMe
Cbz―ΔBut(OMe)―Trp―OMe
and

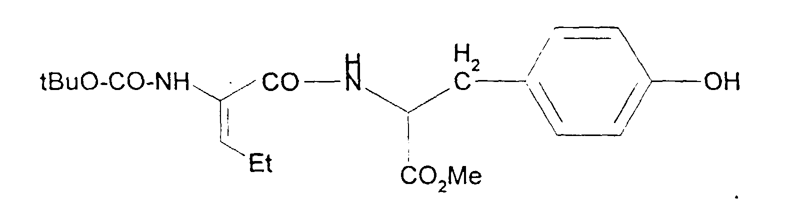

2. Compounds of the formula (I) according to Claim 1 in which
A represents a single bond or alkylene having 1 to 6 carbon atoms which is optionally mono- or polysubstituted by identical or different substituents, the possible substituents being selected from the following list:
halogen, cyano, nitro, amino, hydroxyl, formyl, carboxyl, carbamoyl, thiocarbamoyl;
respectively straight-chain or branched alkoxy, alkylthio, alkylsulphinyl or alkylsulphonyl having in each case 1 to 6 carbon atoms;
respectively straight-chain or branched alkenyl or alkenyloxy having in each case 2 to 6 carbon atoms;
respectively straight-chain or branched halogenoalkoxy, halogenoalkylthio, halogenoalkylsulphinyl or halogenoalkylsulphonyl having in each case 1 to 6 carbon atoms and 1 to 13 identical or different halogen atoms;
respectively straight-chain or branched halogenoalkenyl or halogenoalkenyloxy having in each case 2 to 6 carbon atoms and 1 to 13 identical or different halogen atoms;
respectively straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties;
cycloalkyl having 3 to 6 carbon atoms;
and aryl or heterocyclyl which is in each case optionally mono- or polysubstituted by identical or different substituents from the group consisting of
halogen, cyano and straight-chain or branched alkyl having 1 to 4 carbon atoms
and straight-chain or branched halogenoalkyl having 1 to 4 carbon atoms and 1 to 9 identical or different halogen atoms
and straight-chain or branched alkoxy or alkylthio having 1 to 4 carbon atoms
and straight-chain or branched halogenoalkoxy or halogenoalkylthio having 1 to 4 carbon atoms and 1 to 9 identical or different halogen atoms
and alkylene or dioxyalkylene having in each case 1 to 6 carbon atoms which is in each case attached twice and optionally mono- or polysubstituted by identical or different substituents from the group consisting of halogen and straight-chain or branched alkyl having 1 to 4 carbon atoms and straight-chain or branched halogenoalkyl having 1 to 4 carbon atoms and 1 to 9 identical or different halogen atoms.
Q¹, Q², Q³ are identical or different and each represent oxygen or sulphur,
R¹ represents alkyl having 1 to 8 carbon atoms which is optionally mono- or polysubstituted by identical or different substituents from the group consisting of halogen, cyano, hydroxyl, amino, C₁-C₄-alkoxy, C₁-C₄-alkylthio, C₁-C₄-alkylsulphinyl and C₁-C₄-alkylsulphonyl (each of which may optionally be substituted by halogen);
or represents respectively optionally halogen-substituted alkenyl or alkinyl having in each case up to 8 carbon atoms;
or represents cycloalkyl or cycloalkenyl having 3 to 7 carbon atoms which is respectively optionally mono- or polysubstituted by identical or different substituents from the group consisting of halogen, cyano, carboxyl, phenyl (which is optionally substituted by halogen, cyano, C₁-C₄-alkyl, C₁-C₄-halogenoalkyl, C₁-C₄-alkoxy or C₁-C₄-halogenoalkoxy), C₁-C₄-alkyl and C₁-C₄-alkoxy-carbonyl;
or represents aryl, arylalkyl or heterocyclyl having 3 to 12 ring members which is respectively optionally mono- or polysubstituted by identical or different substituents, the possible substituents being selected from the following list:
halogen, cyano, nitro, amino, hydroxyl, formyl, carboxyl, carbamoyl, thiocarbamoyl;
respectively straight-chain or branched alkyl, alkoxy, alkylthio, alkylsulphinyl or alkylsulphonyl having in each case 1 to 6 carbon atoms;
respectively straight-chain or branched alkenyl or alkenyloxy having in each case 2 to 6 carbon atoms;
respectively straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
respectively straight-chain or branched halogenoalkenyl or halogenoalkenyloxy having in each case 2 to 6 carbon atoms and 1 to 13 identical or different halogen atoms;
respectively straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties;
alkylene or dioxyalkylene having in each case 1 to 6 carbon atoms which is in each case attached twice and in each case optionally mono- or polysubstituted by identical or different substituents from the group consisting of halogen and straight-chain or branched alkyl having 1 to 4 carbon atoms and straight-chain or branched halogenoalkyl having 1 to 4 carbon atoms and 1 to 9 identical or different halogen atoms;
cycloalkyl having 3 to 6 carbon atoms;
and aryl, aryloxy, arylthio, arylalkyl, arylalkyloxy, arylalkylthio, heterocyclyl, heterocyclyloxy, heterocyclylthio, heterocyclylalkyl, heterocyclylalkyloxy or heterocyclylalkylthio,
each of which is optionally mono- or polysubstituted by identical or different substituents from the group consisting of
halogen, cyano and straight-chain or branched alkyl having 1 to 4 carbon atoms
and straight-chain or branched halogenoalkyl having 1 to 4 carbon atoms and 1 to 9 identical or different halogen atoms
and straight-chain or branched alkoxy or alkylthio having 1 to 4 carbon atoms
and straight-chain or branched halogenoalkoxy or halogenoalkylthio having 1 to 4 carbon atoms and 1 to 9 identical or different halogen atoms
and alkylene or dioxyalkylene having in each case 1 to 6 carbon atoms which is in each case attached twice and optionally mono- or polysubstituted by identical or different substituents from the group consisting of halogen and straight-chain or branched alkyl having 1 to 4 carbon atoms and straight-chain or branched halogenoalkyl having 1 to 4 carbon atoms and 1 to 9 identical or different halogen atoms.
R², R⁵ are identical or different and each represent hydrogen or represent alkyl having 1 to 4 carbon atoms or cycloalkyl having 3 to 6 carbon atoms each of which is optionally mono- or polysubstituted by identical or different substituents from the group consisting of halogen, cyano, hydroxyl, amino, C₁-C₄-alkoxy, C₁-C₄-alkylthio, C₁-C₄-alkylsulphinyl and C₁-C₄-alkylsulphonyl (each of which may optionally be substituted by halogen),
R³ represents hydrogen, or represents alkyl having 1 to 8 carbon atoms which is in each case optionally mono- or polysubstituted by identical or different substituents from the group consisting of halogen, cyano, hydroxyl, amino, C₁-C₄-alkoxy, C₁-C₄-alkylthio, C₁-C₄-alkylsulphinyl and C₁-C₄-alkylsulphonyl (each of which may optionally be substituted by halogen);
or represents respectively optionally halogen-substituted alkenyl or alkinyl having in each case up to 8 carbon atoms;
or represents cycloalkyl having 3 to 6 carbon atoms, which is in each case optionally mono- or polysubstituted by identical or different substituents from the group consisting of halogen, cyano, carboxyl, phenyl (which is optionally substituted by halogen, cyano, C₁-C₄-alkyl, C₁-C₄-halogenoalkyl, C₁-C₄-alkoxy or C₁-C₄-halogenoalkoxy), C₁-C₄-alkyl and C₁-C₄-alkoxy-carbonyl,
R⁴ represents alkyl having 1 to 8 carbon atoms which is in each case optionally mono- or polysubstituted by identical or different substituents from the group consisting of halogen, cyano, hydroxyl, amino, C₁-C₄-alkoxy, C₁-C₄-alkylthio, C₁-C₄-alkylsulphinyl and C₁-C₄-alkylsulphonyl (each of which may optionally be substituted by halogen);
or represents respectively optionally halogen-substituted alkenyl or alkinyl having in each case up to 8 carbon atoms;
or represents cycloalkyl having 3 to 6 carbon atoms which is in each case optionally mono- or polysubstituted by identical or different substituents from the group consisting of halogen, cyano, carboxyl, phenyl (which is optionally substituted by halogen, cyano, C₁-C₄-alkyl, C₁-C₄-halogenoalkyl, C₁-C₄-alkoxy or C₁-C₄-halogenoalkoxy), C₁-C₄-alkyl and C₁-C₄-alkoxy-carbonyl, or
R³ and R⁴ together with carbon atom that they are attached to form a carbocyclic ring having 3 to 8 carbon atoms which is optionally mono- or polysubstituted by identical or different substituents from the group consisting of halogen, cyano, hydroxyl, amino, C₁-C₄-alkyl, C₁-C₄-alkoxy, C₁-C₄-alkylthio, C₁-C₄-alkylsulphinyl and C₁-C₄-alkylsulphonyl (each of which may optionally be substituted by halogen) and
R⁶ represents cycloalkyl or cycloalkenyl having 3 to 8 carbon atoms which is in each case optionally mono- or polysubstituted by identical or different substituents from the group consisting of halogen, cyano, carboxyl, phenyl (which is optionally substituted by halogen, cyano, C₁-C₄-alkyl, C₁-C₄-halogenoalkyl, C₁-C₄-alkoxy or C₁-C₄-halogenoalkoxy), C₁-C₄-alkyl and C₁-C₄-alkoxy-carbonyl;
or represents aryl or heterocyclyl having 3 to 12 ring members, each of which is optionally mono- or polysubstituted by identical or different substituents, the possible substituents being selected from the following list:
halogen, cyano, nitro, amino, hydroxyl, formyl, carboxyl, carbamoyl, thiocarbamoyl;
respectively straight-chain or branched alkyl, alkoxy, alkylthio, alkylsulphinyl or alkylsulphonyl having in each case 1 to 6 carbon atoms;
respectively straight-chain or branched alkenyl or alkenyloxy having in each case 2 to 6 carbon atoms;
respectively straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
respectively straight-chain or branched halogenoalkenyl or halogenoalkenyloxy having in each case 2 to 6 carbon atoms and 1 to 13 identical or different halogen atoms;
respectively straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties;
alkylene or dioxyalkylene having in each case 1 to 6 carbon atoms which is in each case attached twice and in each case optionally mono- or polysubstituted by identical or different substituents from the group consisting of halogen and straight-chain or branched alkyl having 1 to 4 carbon atoms and straight-chain or branched halogenoalkyl having 1 to 4 carbon atoms and 1 to 9 identical or different halogen atoms;
cycloalkyl having 3 to 6 carbon atoms;
and aryl, aryloxy, arylthio, arylalkyl, arylalkyloxy, arylalkylthio, heterocyclyl, heterocyclyloxy, heterocyclylthio, heterocyclylalkyl, heterocyclylalkyloxy or heterocyclylalkylthio,
each of which is optionally mono- or polysubstituted by identical or different substituents from the group consisting of
halogen, cyano and straight-chain or branched alkyl having 1 to 4 carbon atoms
and straight-chain or branched halogenoalkyl having 1 to 4 carbon atoms and 1 to 9 identical or different halogen atoms
and straight-chain or branched alkoxy or alkylthio having 1 to 4 carbon atoms
and straight-chain or branched halogenoalkoxy or halogenoalkylthio having 1 to 4 carbon atoms and 1 to 9 identical or different halogen atoms
and alkylene or dioxyalkylene having in each case 1 to 6 carbon atoms which is in each case attached twice and optionally mono- or polysubstituted by identical or different substituents from the group consisting of halogen and straight-chain or branched alkyl having 1 to 4 carbon atoms and straight-chain or branched halogenoalkyl having 1 to 4 carbon atoms and 1 to 9 identical or different halogen atoms.

3. Compounds of the formula (I) according to Claim 1 in which
A represents a single bond or represents methylene, 1,1-ethylene, 1,2-ethylene, 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene), each of which is optionally mono- or polysubstituted by identical or different substituents from the group consisting of fluorine, chlorine, cyano, methoxy, phenyl, tolyl, ethylphenyl, methoxyphenyl, ethoxyphenyl, chlorophenyl, fluorophenyl, trifluoromethylphenyl or xylyl,
Q¹, Q², Q³ are identical or different and each represent oxygen or sulphur,
R¹ represents methyl, ethyl, n- or i-propyl, n-, i-, s- or t-butyl, 1-, 2-, 3-, neo-pentyl, 1-, 2-, 3-, 4-(2-methylbutyl), 1-, 2-, 3-hexyl, 1-, 2-, 3-, 4-, 5-(2-methylpentyl), 1-, 2-, 3-(3-methylpentyl), 2-ethylbutyl, 1-, 3-, 4-(2,2-dimethylbutyl), 1-, 2-(2,3-dimethylbutyl), each of which is optionally mono- to pentasubstituted by fluorine, chlorine, bromine, cyano, hydroxyl, amino, methoxy, ethoxy, methylthio, ethylthio, methylsulphinyl, ethylsulphinyl, methylsulphonyl, ethylsulphonyl (each of which is optionally substituted by fluorine and/or chlorine),
or represents allyl, crotonyl, 1-methyl-allyl, propargyl or 1-methylpropargyl, each of which is optionally mono- to trisubstituted by fluorine, chlorine or bromine;
or represents cyclopropyl, cyclobutyl, cyclopentyl or cyclohexyl, each of which is optionally mono- to hexasubstituted by fluorine, chlorine, bromine, methoxy, ethoxy, cyano, carboxyl, phenyl (which is optionally substituted by fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy), methyl, ethyl, n- or i-propyl, methoxy-carbonyl or ethoxy-carbonyl;
or represents respectively optionally mono- to trisubstituted phenyl, naphthyl, benzyl, phenethyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, the possible substituents being selected from the following list:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, nor i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamine, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl and ethoximinoethyl;
trimethylene (propane-1,3-diyl), methylenedioxy or ethylenedioxy, each of which is attached twice and each of which is optionally mono- or polysubstituted by identical or different substituents from the group consisting of fluorine, chlorine, methyl, trifluoromethyl, ethyl, n- and i-propyl,
cyclopropyl, cyclobutyl, cyclopentyl or cyclohexyl.
R², R⁵ are identical or different and each represent hydrogen or represent methyl, ethyl, n- or i-propyl, n-, i-, s- or t-butyl,
R³ represents hydrogen or represents methyl, ethyl, n- or i-propyl, n-, i-, s-or t-butyl, each of which is optionally mono- to pentasubstituted by fluorine, chlorine, bromine, cyano, hydroxyl, amino, methoxy, ethoxy, methylthio, ethylthio, methylsulphinyl, ethylsulphinyl, methylsulphonyl, ethylsulphonyl (each of which is optionally substituted by fluorine and/or chlorine),
or represents allyl, crotonyl, 1-methyl-allyl, propargyl or 1-methylpropargyl, each of which is optionally mono- to trisubstituted by fluorine, chlorine or bromine;
or represents cyclopropyl, cyclobutyl, cyclopentyl or cyclohexyl, each of which is optionally mono- to hexasubstituted by fluorine, chlorine, bromine, cyano, carboxyl, methyl, ethyl, n- or i-propyl, methoxy-carbonyl or ethoxy-carbonyl,
R⁴ represents methyl, ethyl, n- or i-propyl, n-, i-, s- or t-butyl, each of which is optionally mono- to pentasubstituted by fluorine, chlorine, bromine, cyano, hydroxyl, amino, methoxy, ethoxy, methylthio, ethylthio, methylsulphinyl, ethylsulphinyl, methylsulphonyl, ethylsulphonyl (each of which is optionally substituted by fluorine and/or chlorine),
or represents allyl, crotonyl, 1-methyl-allyl, propargyl or 1-methylpropargyl, each of which is optionally mono- to trisubstituted by fluorine, chlorine or bromine;
or represents cyclopropyl, cyclobutyl, cyclopentyl or cyclohexyl, each of which is optionally mono- to hexasubstituted by fluorine, chlorine, bromine, cyano, carboxyl, methyl, ethyl, n- or i-propyl, methoxy-carbonyl or ethoxy-carbonyl, or
R³ and R⁴ together with the carbon atom that they are attached to represent cyclopropyl, cyclobutyl, cyclopentyl or cyclohexyl, each of which is optionally mono- to hexasubstituted by fluorine, chlorine, bromine, cyano, carboxyl, methyl, ethyl, n- or i-propyl, methoxy-carbonyl or ethoxy-carbonyl and
R⁶ represents cyclopropyl, cyclobutyl, cyclopentyl or cyclohexyl, each of which is optionally mono- to hexasubstituted by fluorine, chlorine, bromine, cyano, carboxyl, methyl, ethyl, methoxy, ethoxy, n- or i-propyl, methoxy-carbonyl or ethoxy-carbonyl;
or represents respectively optionally mono- to trisubstituted phenyl, naphthyl, benzyl, phenethyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, the possible substituents being selected from the following list:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, nor i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamine, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl and ethoximinoethyl;
trimethylene (propane-1,3-diyl), methylenedioxy or ethylenedioxy, each of which is attached twice and each of which is optionally mono- or polysubstituted by identical or different substituents from the group consisting of fluorine, chlorine, methyl, trifluoromethyl, ethyl, n- and i-propyl,
cyclopropyl, cyclobutyl, cyclopentyl or cyclohexyl.

4. Compounds of the formula (I) according to Claim 1 in which
A represents a single bond or represents methylene, 1,1-ethylene, 1,2-ethylene, 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene), each of which is optionally mono- or polysubstituted by identical or different substituents from the group consisting of fluorine, chlorine, cyano, methoxy, phenyl, tolyl, ethylphenyl, methoxyphenyl, ethoxyphenyl, chlorophenyl, fluorophenyl, trifluoromethylphenyl or xylyl,
Q¹, Q², Q³ are identical or different and each represent oxygen or sulphur,
R¹ represents methyl, ethyl, n- or i-propyl, n-, i-, s- or t-butyl, each of which is optionally mono- to trisubstituted by fluorine, chlorine, bromine, methoxy, ethoxy, methylthio, ethylthio (each of which is optionally substituted by fluorine and/or chlorine),
or represents allyl, crotonyl, 1-methyl-allyl, propargyl or 1-methylpropargyl, each of which is optionally mono- to trisubstituted by fluorine or chlorine;
or represents cyclobutyl, cyclopentyl or cyclohexyl, each of which is optionally mono- to hexasubstituted by fluorine, chlorine, bromine, methoxy, ethoxy, phenyl (which is optionally substituted by fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy), methyl, ethyl, n- or i-propyl;
or represents respectively optionally mono- to trisubstituted phenyl, naphthyl, benzyl, furyl, benzofuranyl, thienyl, benzothienyl, pyridyl, quinolyl, tetrahydrofuryl or perhydropyranyl, the possible substituents being selected from the following list:
fluorine, chlorine, bromine, cyano, nitro, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoro-methylthio, trifluoromethylsulphinyl, trifluoromethyl-sulphonyl, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl and ethoximinoethyl;
trimethylene (propane-1,3-diyl), methylenedioxy or ethylenedioxy, each of which is attached twice and each of which is optionally mono- or polysubstituted by identical or different substituents from the group consisting of fluorine, chlorine, methyl, trifluoromethyl, ethyl, n- and i-propyl,
R²,R⁵ are identical or different and each represent hydrogen or represent methyl,
R³ represents hydrogen or represents methyl, ethyl, n- or i-propyl, n-, i-, s-or t-butyl, each of which is optionally mono- to pentasubstituted by fluorine, chlorine, bromine, cyano, hydroxyl, amino, methoxy, ethoxy, methylthio, ethylthio, methylsulphinyl, ethylsulphinyl, methylsulphonyl, ethylsulphonyl (each of which is optionally substituted by fluorine and/or chlorine),
or represents allyl, crotonyl, 1-methyl-allyl, propargyl or 1-methylpropargyl, each of which is optionally mono- to trisubstituted by fluorine, chlorine or bromine;
or represents cyclopropyl, cyclobutyl, cyclopentyl or cyclohexyl, each of which is optionally mono- to hexasubstituted by fluorine, chlorine, bromine, cyano, carboxyl, methyl, ethyl, n- or i-propyl, methoxy-carbonyl or ethoxy-carbonyl,
R⁴ represents methyl, ethyl, n- or i-propyl, n-, i-, s- or t-butyl, each of which is optionally mono- to pentasubstituted by fluorine, chlorine, bromine, cyano, hydroxyl, amino, methoxy, ethoxy, methylthio, ethylthio, methylsulphinyl, ethylsulphinyl, methylsulphonyl, ethylsulphonyl (each of which is optionally substituted by fluorine and/or chlorine),
or represents allyl, crotonyl, 1-methyl-allyl, propargyl or 1-methylpropargyl, each of which is optionally mono- to trisubstituted by fluorine, chlorine or bromine;
or represents cyclopropyl, cyclobutyl, cyclopentyl or cyclohexyl, each of which is optionally mono- to hexasubstituted by fluorine, chlorine, bromine, cyano, carboxyl, methyl, ethyl, n- or i-propyl, methoxy-carbonyl or ethoxy-carbonyl,
R³ and R⁴ together with the carbon atom that they are attached to represent cyclopropyl, cyclobutyl, cyclopentyl or cyclohexyl, each of which is optionally mono- to hexasubstituted by fluorine, chlorine, bromine, cyano, carboxyl, methyl, ethyl, n- or i-propyl, methoxy-carbonyl or ethoxy-carbonyl and
R⁶ represents cyclopropyl, cyclobutyl, cyclopentyl or cyclohexyl, each of which is optionally mono- to hexasubstituted by fluorine, chlorine, bromine, cyano, carboxyl, methyl, ethyl, methoxy, ethoxy, n- or i-propyl, methoxy-carbonyl or ethoxy-carbonyl;
or represents respectively optionally mono- to trisubstituted phenyl, naphthyl, benzyl, phenethyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, the possible substituents being selected from the following list:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, nor i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoro-methylthio, trifluoromethylsulphinyl or trifluoromethyl-sulphonyl, methylamine, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl and ethoximinoethyl;
trimethylene (propane-1,3-diyl), methylenedioxy or ethylenedioxy, each of which is attached twice and each of which is optionally mono- or polysubstituted by identical or different substituents from the group consisting of fluorine, chlorine, methyl, trifluoromethyl, ethyl, n- and i-propyl,
cyclopropyl, cyclobutyl, cyclopentyl or cyclohexyl.

5. Compounds of the formula (I) according to Claim 1 in which
A represents a single bond or represents methylene, 1,1-ethylene, 1,2-ethylene, 1,1-propylene, 1,2-propylene or 2,2-propylene,
Q¹, Q², Q³ each represent oxygen,
R¹ represents methyl, ethyl, n- or i-propyl, n-, i-, s- or t-butyl, cyclopentyl, cyclohexyl or represents optionally methyl-, ethyl-, methoxy-, ethoxy-, bromine-, chlorine- or fluorine-substituted phenyl or benzyl,
R², R⁵ each represent hydrogen,
R³ represents hydrogen, methyl, ethyl or cyclopropyl,
R⁴ represents methyl, ethyl or cyclopropyl, or
R³ and R⁴ together with the carbon atom that they are attached to form a cyclopentane or cyclohexane ring and
R⁶ represents cyclohexyl which is in each case optionally mono- or disubstituted by methyl, ethyl, methoxy or ethoxy;
or represents phenyl, naphthyl, furyl, benzofuranyl, pyrrolyl, indolyl, thienyl, benzothienyl, pyridyl, quinolyl, pyrimidyl, pyridazinyl, pyrazinyl, each of which is optionally mono- to trisubstituted, the possible substituents being selected from the following list:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, nor i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamine, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl and ethoximinoethyl;
trimethylene (propane-1,3-diyl), methylenedioxy or ethylenedioxy, each of which is attached twice and each of which is optionally mono- or polysubstituted by identical or different substituents from the group consisting of fluorine, chlorine, methyl, trifluoromethyl, ethyl, n- and i-propyl.

6. Pesticides, characterized by a content of at least one compound of the formula (I) according to Claim 1.

7. Method for controlling pests, characterized in that compounds of the formula (I) according to Claim 1 are allowed to act on pests and/or their habitat.

8. Use of compounds of the formula (I) according to any of Claims 1 to 5 for controlling pests.

9. Process for preparing pesticides, characterized in that compounds of the formula (I) according to any of Claims 1 to 5 are mixed with extenders and/or surface-active agents.

10. Process for preparing compounds of the formula (I) according to Claim 1, characterized in that N-substituted amino acids of the general formula (II), 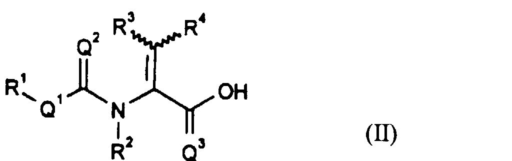 in which
Q¹, Q², Q³, R¹, R², R³ and R⁴ are each as defined in Claim 1,
are reacted with amines of the general formula (III), 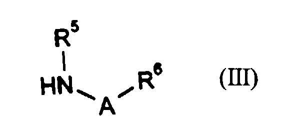 in which
A, R⁵ and R⁶ are each as defined in Claim 1,
if appropriate in the presence of a reaction auxiliary and if appropriate in the presence of a diluent.

## Revendications

1. Composés de formule (I) 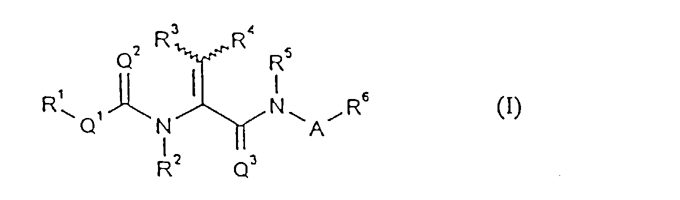 dans laquelle
A est une liaison simple ou un groupe alkylène éventuellement substitué,
Q¹, Q², Q³ sont identiques ou différents et représentent l'oxygène ou le soufre,
R¹ est un groupe, éventuellement substitué dans chaque cas, alkyle, alcényle, alcynyle, cycloalkyle, cycloalcényle, arylalkyle, aryle, hétérocyclylalkyle ou hétérocyclyle,
R², R⁵ sont identiques ou différents et représentent chacun l'hydrogène ou un groupe alkyle éventuellement substitué,
R³ est l'hydrogène, un groupe alkyle ou cycloalkyle dont chacun est éventuellement substitué,
R⁴ est un groupe alkyle ou un groupe cycloalkyle dont chacun est éventuellement substitué ou bien
R³ et R⁴ forment conjointement avec l'atome de carbone auquel ils sont liés un noyau carbocyclique éventuellement substitué et
R⁶ est un groupe cycloalkyle, cycloalcényle, aryle ou hétérocyclyle, dont chacun est éventuellement substitué,
à l'exception des composés
Cbz―ΔGlu(OMe)―Phe―NHPh
Cbz―ΔGlu(OMe)―Try―NHPh
Cbz―ΔGlu(OMe)―NHPh
Cbz―ΔGln(Bu^{t})―Phe―OMe
Boc―ΔnVal(OMe)―L-Tyr―OMe
Cbz―ΔBut(OMe)―Phe―OEt
Cbz―ΔBut(OMe)―Tyr―OMe
Cbz―ΔBut(OMe)―Trp―OMe
et

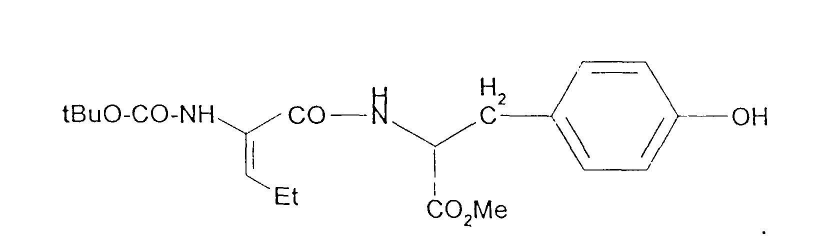

2. Composés de formule (I) suivant la revendication 1, dans laquelle
A est une liaison simple ou un groupe alkylène en C₁ à C₆ portant le cas échéant un ou plusieurs substituants identiques ou différents, les substituants possibles étant choisis dans l'énumération suivante :
halogène, cyano, nitro, amino, hydroxy, formyle, carboxy, carbamoyle, thiocarbamoyle ;
alkoxy, alkylthio, alkylsulfinyle ou alkylsulfonyle ayant chacun 1 à 6 atomes de carbone et étant chacun linéaire ou ramifié ;
alcényle ou alcényloxy ayant chacun 2 à 6 atomes de carbone et étant chacun linéaire ou ramifié ;
halogénalkoxy, halogénalkylthio, halogénalkylsulfinyle ou halogénalkylsulfonyle ayant chacun 1 à 6 atomes de carbone et 1 à 13 atomes d'halogènes identiques ou différents et étant chacun linéaire ou ramifié ;
halogénalcényle ou halogénalcényloxy ayant chacun 2 à 6 atomes de carbone et 1 à 13 atomes d'halogènes identiques ou différents et étant chacun linéaire ou ramifié ;
alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles et étant chacun linéaire ou ramifié ;
cycloalkyle ayant 3 à 6 atomes de carbone ;
de même qu'aryle ou hétérocyclyle substitué le cas échéant une ou plusieurs fois identiques ou différentes par
halogène, cyano et/ou alkyle linéaire ou ramifié ayant 1 à 4 atomes de carbone
et/ou halogénalkyle linéaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents
et/ou alkylène ou dioxyalkylène ayant chacun 1 à 6 atomes de carbone, chacun deux liaisons, et substitués chacun le cas échéant une ou plusieurs fois identiques ou différentes par un halogène et/ou un radical alkyle linéaire ou ramifié ayant 1 à 4 atomes de carbone et/ou
halogénalkyle linéaire ou ramifié ayant 1 à 4 atomes de carbone et 1 à 9 atomes d'halogènes identiques ou différents,
Q¹, Q², Q³ sont identiques ou différents et représentent chacun l'oxygène ou le soufre,
R¹ est un groupe alkyle ayant 1 à 8 atomes de carbone portant éventuellement un ou plusieurs substituants, identiques ou différents, halogéno, cyano, hydroxy, amino, alkoxy en C₁ à C₄, alkylthio en C₁ à C₄, alkylsulfinyle en C₁ à C₄, alkylsulfonyle en C₁ à C₄ (qui peuvent tous être substitués le cas échéant par un halogène) ;
un groupe alcényle ou alcynyle ayant chacun jusqu'à 8 atomes de carbone et substitués chacun le cas échéant par un halogène ;
un groupe cycloalkyle ou cycloalcényle ayant 3 à 7 atomes de carbone, chacun étant substitué le cas échéant une ou plusieurs fois identiques ou différentes par un radical halogéno, cyano, carboxy, phényle (qui est éventuellement substitué par un radical halogéno, cyano, alkyle en C₁ à C₄, halogénalkyle en C₁ à C₄, alkoxy en C₁ à C₄ ou halogénalkoxy en C₁ à C₄), alkyle en C₁ à C₄ ou (alkoxy en C₁ à C₄)-carbonyle, un groupe aryle, arylalkyle ou un groupe hétérocyclyle à noyau de 3 à 12 atomes, chacun étant substitué le cas échéant une ou plusieurs fois identiques ou différentes, les substituants possibles étant choisis dans les énumérations suivantes :
halogène, cyano, nitro, amino, hydroxy, formyle, carboxy, carbamoyle, thiocarbamoyle ;
alkyle, alkoxy, alkylthio, alkylsulfinyle ou alkylsulfonyle ayant chacun 1 à 6 atomes de carbone et étant chacun linéaire ou ramifié ;
alcényle ou alcényloxy ayant chacun 2 à 6 atomes de carbone et étant chacun linéaire ou ramifié ;
halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents et étant chacun linéaire ou ramifié ;
halogénalcényle ou halogénalcényloxy ayant chacun 2 à 6 atomes de carbone et 1 à 13 atomes d'halogènes identiques ou différents et étant chacun linéaire ou ramifié ;
alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles et étant chacun linéaire ou ramifié ;
alkylène ou dioxyalkylène ayant chacun 1 à 6 atomes de carbone, chacun deux liaisons, et portant chacun le cas échéant un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 6 atomes d'halogènes identiques ou différents ;
cycloalkyle ayant 3 à 6 atomes de carbone ;
ainsi qu'aryle, aryloxy, arylthio, arylalkyle, arylalkyloxy, arylalkylthio, hétérocyclyle, hétérocyclyloxy, hétérocyclylthio, hétérocyclylalkyle, hétérocyclylalkyloxy ou hétérocyclylalkylthio portant chacun le cas échéant un ou plusieurs substituants, identiques ou différents,
halogéno, cyano et/ou alkyle linéaire ou ramifié ayant 1 à 4 atomes de carbone,
et/ou halogénalkyle linéaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone,
et/ou halogénalkoxy ou halogénalkylthio linéaire ou ramifié ayant 1 à 4 atomes de carbone et 1 à 9 atomes d'halogènes identiques ou différents
et/ou alkylène ou dioxyalkylène ayant chacun 1 à 6 atomes de carbone, chacun deux liaisons et portant le cas échéant un ou plusieurs substituants, identiques ou différents, halogéno et/ou
alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
R², R⁵ sont identiques ou différents et représentent chacun l'hydrogène ou un groupe alkyle ayant 1 à 4 atomes de carbone ou cycloalkyle ayant 3 à 6 atomes de carbone, chacun étant substitué le cas échéant une ou plusieurs fois identiques ou différentes par un radical halogéno, cyano, hydroxy, amino, alkoxy en C₁ à C₄ alkylthio en C₁ à C₄, alkylsulfinyle en C₁ à C₄ ou alkylsulfonyle en C₁ à C₄ (chacun d'eux pouvant éventuellement être substitué par un halogène),
R³ représente l'hydrogène, un groupe alkyle ayant 1 à 8 atomes de carbone portant le cas échéant un ou plusieurs substituants, identiques ou différents, halogéno, cyano, hydroxy, amino, alkoxy en C₁ à C₄, alkylthio en C₁ à C₄, alkylsulfinyle en C₁ à C₄ ou alkylsulfonyle en C₁ à C₄ (chacun d'eux pouvant éventuellement être substitué par un halogène) ;
un groupe alcényle ou alcynyle ayant chacun jusqu'à 8 atomes de carbone et chacun étant éventuellement substitué par un halogène ;
un groupe cycloalkyle ayant 3 à 6 atomes de carbone portant le cas échéant un ou plusieurs substituants, identiques ou différents, halogéno, cyano, carboxy, phényle (qui est substitué le cas échéant par un radical halogéno, cyano, alkyle en C₁ à C₄, halogénalkyle en C₁ à C₄, alkoxy en C₁ à C₄ ou halogénalkoxy en C₁ à C₄), alkyle en C₁ à C₄ ou (alkoxy en C₁ à C₄)-carbonyle,
R⁴ est un groupe alkyle ayant 1 à 8 atomes de carbone portant le cas échéant un ou plusieurs substituants, identiques ou différents, halogéno, cyano, hydroxy, amino, alkoxy en C₁ à C₄, alkylthio en C₁ à C₄, alkylsulfinyle en C₁ à C₄ ou alkylsulfonyle en C₁ à C₄ (chacun d'eux pouvant éventuellement être substitué par un halogène) ;
un groupe alcényle ou alcynyle ayant chacun jusqu'à 8 atomes de carbone et chacun étant éventuellement substitué par un halogène ;
un groupe cycloalkyle ayant 3 à 6 atomes de carbone substitué le cas échéant une ou plusieurs fois identiques ou différentes par un radical halogéno, cyano, carboxy, phényle (qui est substitué le cas échéant par un radical halogéno, cyano, alkyle en C₁ à C₄, halogénalkyle en C₁ à C₄, alkoxy en C₁ à C₄ ou halogénalkoxy en C₁ à C₄), alkyle en C₁ à C₄ ou (alkoxy en C₁ à C₄)-carbonyle, ou bien
R³ et R⁴ forment conjointement avec l'atome de carbone auquel ils sont liés un noyau carbocyclique de 3 à 8 atomes de carbone portant le cas échéant un ou plusieurs substituants, identiques ou différents, halogéno, cyano, hydroxy, amino, alkyle en C₁ à C₄, alkoxy en C₁ à C₄, alkylthio en C₁ à C₄, alkylsulfinyle en C₁ à C₄ ou alkylsulfonyle en C₁ à C₄ (chacun pouvant être substitué le cas échéant par un halogène) et
R⁶ est un groupe cycloalkyle ou cycloalcényle de 3 à 8 atomes de carbone, chacun étant substitué le cas échéant une ou plusieurs fois identiques ou différentes par un radical halogéno, cyano, carboxy, phényle (qui est substitué le cas échéant par un radical halogéno, cyano, alkyle en C₁ à C₄, halogénalkyle en C₁ à C₄, alkoxy en C₁ à C₄ ou halogénalkoxy en C₁ à C₄), alkyle en C₁ à C₄ ou (alkoxy en C₁ à C₄)-carbonyle ;
un groupe aryle ou un groupe hétérocyclyle à noyau de 3 à 12 atomes, chacun étant substitué le cas échéant une ou plusieurs fois identiques ou différentes, les substituants possibles étant choisis dans l'énumération suivante :
halogène, cyano, nitro, amino, hydroxy, formyle, carboxy, carbamoyle, thiocarbamoyle ;
alkyle, alkoxy, alkylthio, alkylsulfinyle ou alkylsulfonyle ayant chacun 1 à 6 atomes de carbone et étant chacun linéaire ou ramifié ;
alcényle ou alcényloxy ayant chacun 2 à 6 atomes de carbone et étant chacun linéaire ou ramifié ;
halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents et étant chacun linéaire ou ramifié ;
halogénalcényle ou halogénalcényloxy ayant chacun 2 à 6 atomes de carbone et 1 à 13 atomes d'halogènes identiques ou différents et étant chacun linéaire ou ramifié ;
alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles et étant chacun linéaire ou ramifié ;
alkylène ou dioxyalkylène ayant chacun 1 à 6 atomes de carbone, chacun deux liaisons et portant chacun le cas échéant un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
ainsi qu'aryle, aryloxy, arylthio, arylalkyle, arylalkyloxy, arylalkylthio, hétérocyclyle, hétérocyclyloxy, hétérocyclylthio, hétérocyclylalkyle, hétérocyclylalkyloxy ou hétérocyclylalkylthio portant chacun le cas échéant un ou plusieurs substituants, identiques ou différents,
halogéno, cyano et/ou alkyle linéaire ou ramifié ayant 1 à 4 atomes de carbone,
et/ou halogénalkyle linéaire ou ramifié ayant chacun 1 à 4 atomes de carbone et 1 à 9 atomes d'halogènes identiques ou différents
et/ou alkoxy ou alkylthio linéaire ou ramifié ayant 1 à 4 atomes de carbone,
et/ou halogénalkoxy ou halogénalkylthio linéaire ou ramifié ayant 1 à 4 atomes de carbone et 1 à 9 atomes d'halogènes identiques ou différents
et/ou alkylène ou dioxyalkylène ayant chacun 1 à 6 atomes de carbone, chacun deux liaisons, et étant substitués chacun une ou plusieurs fois identiques ou différentes par un halogène et/ou un radical alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents.

3. Composés de formule (I) suivant la revendication 1, dans laquelle
A est une liaison simple ou un groupe méthylène, 1,1-éthylène, 1,2-éthylène, 1,1-, 1,2-, 1,3- ou 2,2-propylène, 1,1-, 1,2-, 1,3-, 1,4-, 2,2-, 2,3-butylène ou 1,1-, 1,2- ou 1,3-(2-méthylpropylène) portant éventuellement un ou plusieurs substituants, identiques ou différents, fluor, chlore, cyano, méthoxy, phényle, tolyle, éthylphényle, méthoxyphényle, éthoxyphényle, chlorophényle, fluorophényle, trifluorométhylphényle ou xylyle,
Q¹, Q², Q³ sont identiques ou différents et représentent chacun l'oxygène ou le soufre,
R¹ est un groupe méthyle, éthyle, n-propyle, isopropyle, n-butyle, isobutyle, sec.-butyle, tertio-butyle, 1-, 2-, 3-néopentyle, 1-, 2-, 3-, 4-(2-méthylbutyle), 1-, 2-, 3-hexyle, 1-, 2-, 3-, 4-, 5-(2-méthylpentyle), 1-, 2-, 3-(3-méthylpentyle), 2-éthylbutyle, 1-, 3-, 4-(2,2-diméthylbutyle), 1-,2-(2,3-diméthylbutyle) portant éventuellement 1 à 5 substituants fluoro, chloro, bromo, cyano, hydroxy, amino, méthoxy, éthoxy, méthylthio, éthylthio, méthylsulfinyle, éthylsulfinyle, méthylsulfonyle, éthylsulfonyle (qui sont substitués chacun le cas échéant par du fluor et/ou du chlore) ;
un groupe allyle, crotonyle, 1-méthylallyle, propargyle ou 1-méthylpropargyle portant chacun le cas échéant 1 à 3 substituants fluoro, chloro ou bromo ;
un groupe cyclopropyle, cyclobutyle, cyclopentyle ou cyclohexyle portant chacun le cas échéant 1 à 6 substituants fluoro, chloro, bromo, méthoxy, éthoxy, cyano, carboxy, phényle (qui est substitué le cas échéant par du fluor, du chlore, du brome, un radical cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy), méthyle, éthyle, n-propyle, isopropyle, méthoxycarbonyle ou éthoxycarbonyle ;
un groupe phényle, naphtyle, benzyle, phénéthyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle portant chacun le cas échéant 1 à 3 substituants, les substituants possibles étant choisis dans l'énumération suivante :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle ou trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle ou éthoximinoéthyle ;
triméthylène (propane-1,3-diyle), méthylènedioxy ou éthylènedioxy ayant chacun deux liaisons et portant chacun le cas échéant un ou plusieurs substituants, identiques ou différents, fluoro, chloro, méthyle, trifluorométhyle, éthyle, n-propyle ou isopropyle,
cyclopropyle, cyclobutyle, cyclopentyle ou cyclohexyle.
R², R⁵ sont identiques ou différents et représentent chacun un groupe méthyle, éthyle, n-propyle, isopropyle, n-butyle, isobutyle, sec.-butyle ou tertio-butyle,
R³ est l'hydrogène ou un groupe méthyle, éthyle, n-propyle, isopropyle, n-butyle, isobutyle, sec.-butyle ou tertio-butyle, chacun portant le cas échéant 1 à 5 substituants fluoro, chloro, bromo, cyano, hydroxy, amino, méthoxy, éthoxy, méthylthio, éthylthio, méthylsulfinyle, éthylsulfinyle, méthylsulfonyle, éthylsulfonyle (chacun étant substitué le cas échéant par du fluor et/ou du chlore,
un groupe allyle, crotonyle, 1-méthylallyle, porpargyle ou 1-méthylpropargyle portant chacun le cas échéant 1 à 3 substituants fluoro, chloro ou bromo ;
un groupe cyclopropyle, cyclobutyle, cyclopentyle ou cyclohexyle portant chacun le cas échéant 1 à 6 substituants fluoro, chloro, bromo, cyano, carboxy, méthyle, éthyle, n-propyle, isopropyle, méthoxycarbonyle ou éthoxycarbonyle,
R⁴ est un groupe méthyle, éthyle, n-propyle, isopropyle, n-butyle, isobutyle, sec.-butyle ou tertio-butyle, portant chacun le cas échéant 1 à 5 substituants fluoro, chloro, bromo, cyano, hydroxy, amino, méthoxy, éthoxy, méthylthio, éthylthio, méthylsulfinyle, éthylsulfinyle, méthylsulfonyle, éthylsulfonyle (chacun étant substitué le cas échéant par du fluor et/ou du chlore) ;
un groupe allyle, crotonyle, 1-méthylallyle, propargyle ou 1-méthylpropargyle, chacun étant substitué le cas échéant 1 à 3 fois par du fluor, du chlore ou du brome ;
un groupe cyclopropyle, cyclobutyle, cyclopentyle ou cyclohexyle, chacun étant substitué le cas échéant 1 à 6 fois par du fluor, du chlore, du brome, un radical cyano, carboxy, méthyle, éthyle, n-propyle, isopropyle, méthoxycarbonyle ou éthoxycarbonyle ; ou bien
R³ et R⁴ forment conjointement avec l'atome de carbone auquel ils sont liés un groupe cyclopropyle, cyclobutyle, cyclopentyle ou cyclohexyle portant chacun le cas échéant 1 à 6 substituants fluoro, chloro, bromo, cyano, carboxy, méthyle, éthyle, n-propyle, isopropyle, méthoxycarbonyle ou éthoxycarbonyle et
R⁶ est un groupe cyclopropyle, cyclobutyle, cyclopentyle ou cyclohexyle portant chacun le cas échéant 1 à 6 substituants fluoro, chloro, bromo, cyano, carboxy, méthyle, éthyle, méthoxy, éthoxy, n-propyle, isopropyle, méthoxycarbonyle ou éthoxycarbonyle ;
un groupe phényle, naphtyle, benzyle, phénéthyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle portant chacun le cas échéant un à trois substituants, les substituants possibles étant choisis dans l'énumération suivante :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle ou éthoximinoéthyle,
triméthylène (propane-1,3-diyle), méthylènedioxy ou éthylènedioxy ayant chacun deux liaisons et portant chacun le cas échéant un ou plusieurs substituants, identiques ou différents, fluoro, chloro, méthyle, trifluorométhyle, éthyle, n-propyle ou isopropyle,
cyclopropyle, cyclobutyle, cyclopentyle ou cyclohexyle.

4. Composés de formule (I) suivant la revendication 1, dans laquelle
A représente une liaison simple ou un groupe méthylène, 1,1-éthylène, 1,2-éthylène, 1,1-, 1,2-, 1,3-, ou 2,2-propylène, 1,1-, 1,2-, 1,3-, 1,4-, 2,2-, 2,3-butylène ou 1,1-, 1,2- ou 1,3-(2-méthylpropylène) portant le cas échéant un ou plusieurs substituants, identiques ou différents, fluoro, chloro, cyano, méthoxy, phényle, tolyle, éthylphényle, méthoxyphényle, éthoxyphényle, chlorophényle, fluorophényle, trifluorométhylphényle ou xylyle,
Q¹, Q², Q³ sont identiques ou différents et représentent chacun l'oxygène ou le soufre,
R¹ est un groupe méthyle, éthyle, n-propyle, isopropyle, n-butyle, isobutyle, sec.-butyle ou tertio-butyle portant le cas échéant 1 à 3 substituants fluoro, chloro, bromo, méthoxy, éthoxy, méthylthio, éthylthio, (qui sont substitués chacun le cas échéant par du fluor et/ou du chlore),
un groupe allyle, crotonyle, 1-méthylallyle, propargyle ou 1-méthylpropargyle portant chacun le cas échéant 1 à 3 substituants fluoro ou chloro,
un groupe cyclobutyle, cyclopentyle ou cyclohexyle portant chacun le cas échéant un à six substituants fluoro, chloro, bromo, méthoxy, éthoxy, phényle (qui est substitué le cas échéant par du fluor, du chlore, du brome, un radical cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy, ou trifluorométhoxy) ;
un groupe phényle, naphtyle, benzyle, furyle, benzofurannyle, thiényle, benzothiényle, pyridyle, quinolyle, tétrahydrofuryle ou perhydropyrannyle portant chacun le cas échéant un à trois substituants, les substituants possibles étant choisis dans l'énumération suivante :
fluor, chlore, brome, cyano, nitro, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle ou éthoximinoéthyle ;
triméthylène (propane-1,3-diyle), méthylènedioxy ou éthylènedioxy ayant chacun deux liaisons, portant chacun le cas échéant un ou plusieurs substituants, identiques ou différents, fluoro, chloro, méthyle, trifluorométhyle, éthyle, n-propyle ou isopropyle,
R², R⁵ sont identiques ou différents et représentent chacun l'hydrogène ou un groupe méthyle,
R³ représente l'hydrogène ou un groupe méthyle, éthyle, n-propyle, isopropyle, n-butyle, isobutyle, sec.-butyle ou tertio-butyle, portant chacun le cas échéant un à cinq substituants fluoro, chloro, bromo, cyano, hydroxy, amino, méthoxy, éthoxy, méthylthio, éthylthio, méthylsulfinyle, éthylsulfinyle, méthylsulfonyle, éthylsulfonyle (chacun étant substitué le cas échéant par du fluor et/ou du chlore),
un groupe allyle, crotonyle, 1-méthylallyle, propargyle ou 1-méthylpropargyle portant chacun le cas échéant un à trois substituants fluoro, chloro ou bromo ;
un groupe cyclopropyle, cyclobutyle, cyclopentyle ou cyclohexyle, portant chacun le cas échéant 1 à 6 substituants fluoro, chloro, bromo, cyano, carboxy, méthyle, éthyle, n-propyle, isopropyle, méthoxycarbonyle ou éthoxycarbonyle ;
R⁴ est un groupe méthyle, éthyle, n-propyle, isopropyle, n-butyle, isobutyle, sec.-butyle ou tertio-butyle, portant chacun le cas échéant 1 à 5 substituants fluoro, chloro, bromo, cyano, hydroxy, amino, méthoxy, éthoxy, méthylthio, éthylthio, méthylsulfinyle, éthylsulfinyle, méthylsulfonyle, éthylsulfonyle (chacun étant substitué le cas échéant par du fluor et/ou du chlore) ;
un groupe allyle, crotonyle, 1-méthylallyle, propargyle ou 1-méthylpropargyle, chacun étant substitué le cas échéant 1 à 3 fois par du fluor, du chlore ou du brome ;
un groupe cyclopropyle, cyclobutyle, cyclopentyle ou cyclohexyle, chacun étant substitué le cas échéant 1 à 6 fois par du fluor, du chlore, du brome, un radical cyano, carboxy, méthyle, éthyle, n-propyle, isopropyle, méthoxycarbonyle ou éthoxycarbonyle ;
R³ et R⁴ forment conjointement avec l'atome de carbone auquel ils sont liés un groupe cyclopropyle, cyclobutyle, cyclopentyle ou cyclohexyle substitué le cas échéant 1 à 6 fois par du fluor, du chlore, du brome, un radical cyano, carboxy, méthyle, éthyle, n-propyle, isopropyle, méthoxycarbonyle ou éthoxycarbonyle et
R⁶ est un groupe cyclopropyle, cyclobutyle, cyclopentyle ou cyclohexyle, chacun portant le cas échéant 1 à 6 substituants fluoro, chloro, bromo, cyano, carboxy, méthyle, éthyle, méthoxy, éthoxy, n-propyle, isopropyle, méthoxycarbonyle ou éthoxycarbonyle ;
un groupe phényle, naphtyle, benzyle, phénéthyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle portant chacun le cas échéant 1 à 3 substituants, les substituants possibles étant choisis dans l'énumération suivante :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthyl-sulfonyloxy, éthylsulfonyloxy, hydroximinométhyle, hydroximinoéthyle, méthoximinométhyle, éthoximinométhyle, méthoximinoéthyle, ou éthoximinoéthyle,
triméthylène (propane-1,3-diyle), méthylènedioxy ou éthylènedioxy ayant chacun deux liaisons et portant chacun le cas échéant un ou plusieurs substituants, identiques ou différents, fluoro, chloro, méthyle, trifluorométhyle, éthyle, n-propyle ou isopropyle,
cyclopropyle, cyclobutyle, cyclopentyle ou cyclohexyle.

5. Composés de formule (I) suivant la revendication 1, dans laquelle
A représente une liaison simple ou un groupe méthylène, 1,1-éthylène, 1,2-éthylène, 1,1-propylène, 1,2-propylène ou 2,2-propylène,
Q¹, Q², Q³ représentent de l'oxygène,
R¹ est un groupe méthyle, éthyle, n-propyle, isopropyle, n-butyle, isobutyle, sec.-butyle, tertio-butyle, cyclopentyle, cyclohexyle ou un groupe phényle ou benzyle éventuellement substitué par un radical méthyle, éthyle, méthoxy, éthoxy, bromo, chloro ou fluoro,
R², R⁵ représentent de l'hydrogène,
R³ est de l'hydrogène, un groupe méthyle, éthyle, ou cyclopropyle,
R⁴ est un groupe méthyle, éthyle ou cyclopropyle, ou bien
R³ et R⁴ forment conjointement avec l'atome de carbone auquel ils sont liés un noyau cyclopentane ou cyclohexane et
R⁶ représente un groupe cyclohexyle portant le cas échéant un ou deux substituants méthyle, éthyle, méthoxy ou éthoxy ;
un groupe phényle, naphtyle, furyle, benzofurannyle, pyrrolyle, indolyle, thiényle, benzothiényle, pyridyle, quinolyle, pyrimidyle, pyridazinyle, pyrazinyle, portant chacun le cas échéant un à trois substituants, les substituants possibles étant choisis dans l'énumération suivante :
fluor, chlor, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle ou éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle ou trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle ou éthoximinoéthyle,
un groupe triméthylène (propane-1,3-diyle), méthylènedioxy ou éthylènedioxy ayant chacun deux liaisons et portant chacun le cas échéant un ou plusieurs substituants, identiques ou différents, fluoro, chloro, méthyle, trifluorométhyle, éthyle, n-propyle ou isopropyle.

6. Composition pesticide, caractérisée par une teneur en au moins un composé de formule (I) suivant la revendication 1.

7. Procédé pour combattre des parasites, caractérisé en ce qu'on fait agir des composés de formule (I) suivant la revendication 1 sur des parasites et/ou sur leur milieu.

8. Utilisation de composés de formule (I) suivant les revendications 1 à 5 pour combattre des parasites.

9. Procédé de préparation de compositions pesticides, caractérisé en ce qu'on mélange des composés de formule (I) suivant les revendications 1 à 5 avec des diluants et/ou des agents tensio-actifs.

10. Procédé de préparation de composés de formule (I) suivant la revendication 1, caractérisé en ce qu'on fait réagir des aminoacides N-substitués de formule générale (II) 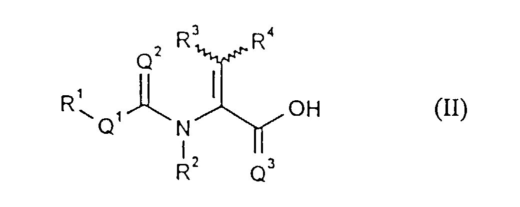 dans laquelle
Q¹, Q², Q³, R¹, R², R³ et R⁴ ont les définitions indiquées dans la revendication 1,
avec des amines de formule générale (III) 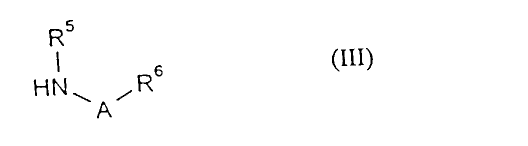 dans laquelle
A, R⁵ et R⁶ ont les définitions indiquées dans la revendication 1,
le cas échéant en présence d'une substance auxiliaire de réaction et en la présence éventuelle d'un diluant.
